# EUROPÄISCHE PATENTANMELDUNG

(11) **EP 4 613 249 A1**
(43) Veröffentlichungstag der Anmeldung: **10.09.2025**
(21) Anmeldenummer: 25160766.9
(22) Anmeldetag: 27.02.2025
(51) Int. Cl.: A61F 13/01, A61L 15/26, A61L 15/28, A61L 15/46

(54) **WUNDKONTAKTSCHICHT MIT INFEKTIONSHEMMENDEN EIGENSCHAFTEN**

(30) Priorität: 07.03.2024 DE 102024106518
(71) Anmelder: Paul Hartmann AG, 89522 Heidenheim (DE)
(72) Erfinder: KETTEL, Markus, 89520 Heidenheim (DE); NOSWORTHY, Jonathan, 89311 Neu-Ulm (DE); Law, Stephen, Liverpool, L7 8XZ (GB); Percival, Steven, Liverpool, L7 8XZ (GB); Chen, Rui, Liverpool, L7 8XZ (GB)
(74) Vertreter: Paul Hartmann AG Patents & Licensing

(57) **Zusammenfassung**

Die vorliegende Erfindung betrifft eine Wundkontaktschicht, welche für die Wundversorgung geeignet ist. Die Wundkontaktschicht umfasst ein netzförmiges Substrat, welches mit einer Emulsion teilweise oder vollständig beschichtet ist, wobei die Emulsion eine antimikrobielle Zusammensetzung enthaltend a) ionisches Silber und / oder Silbernitrat, b) ionisches Zink und / oder Zinknitrat, und c) EDTA umfasst. Die Wundkontaktschicht entfaltet eine antimikrobielle Wirkung und ist gegenüber Biofilmen wirksam. Die Wundkontaktschicht ermöglicht eine Wundpflege und fördert die Wundheilung.

## Beschreibung

### Technisches Gebiet der Erfindung

Die vorliegende Erfindung betrifft eine Wundkontaktschicht umfassend ein netzförmiges Substrat, welches zwei Seiten hat. Die erste Seite des Substrats ist derart beschaffen, dass diese Seite auf eine Wunde aufgelegt werden kann. Mindestens auf dieser ersten Seite befindet sich eine Emulsion, die eine antimikrobielle Zusammensetzung enthält. Diese Zusammensetzung enthält als Wirkstoffe sowohl Silber- als auch Zinkionen und EDTA. Die Wundkontaktschicht eignet sich insbesondere zur Abdeckung infizierter Wunden. Die Salbe verleiht der Wundkontaktschicht zum einen atraumatische Eigenschaften, zum anderen sorgt sie für eine langfristige und gleichmäßige Abgabe der enthaltenen Wirkstoffe an die Wunde.

### Hintergrund der Erfindung

Chronische Wunden stellen in der modernen Medizin nach wie vor ein Problem dar. Gerade bei älteren Menschen und Risikopatienten wie beispielsweise Diabetikern besteht ein erhöhtes Risiko, dass Verletzungen nicht oder nicht vollständig abheilen. Der Wundheilungsprozess ist in einem solchen Fall aus unterschiedlichen Gründen gestört und es besteht ein fortwährender Defekt der Hautbarriere. Dies geht zum einen mit einer Reduktion der Lebensqualität für die Betroffenen einher. Zum anderen erhöht sich mit fortwährender Dauer der Gewebeexposition zunehmend das Risiko einer Infektion. Tritt eine solche Infektion ein, verschlechtert sich die Prognose weiter. Da die körpereigenen Stoffwechselprozesse im Bereich der chronischen Wunde beeinträchtigt sind und eine reguläre Immunantwort nicht oder nicht vollständig erfolgt, kann es in der Folge zu einer Vermehrung der Erreger an der Infektionsstelle kommen. Im weiteren Verlauf bildet sich häufig ein Biofilm, bei dem bakterielle Erreger sich zu einer Lebensgemeinschaft vereinen, die erhöhte Resistenz gegenüber Bioziden und Antibiotika zeigt und daher die weitere Behandlung außerordentlich erschwert. Dieses Stadium erhöht die Wahrscheinlichkeit für weitere Komplikationen wie beispielsweise Nekrosenbildung oder Sepsis.

Aus dem Stand der Technik sind Wundauflagen mit antibiotischer Wirksamkeit bekannt. Diese sind jedoch entweder unzureichend wirksam gegenüber Biofilmen, haben keine wundheilende Wirkung oder zeigen beim Verbandswechsel traumatische Eigenschaften, da sie sich mit Wundbestandteilen verbinden.

Die EP 1 755 569 B9 beschreibt eine Wundauflage mit Salbe, die zusätzlich ein antibakterielles Metall wie beispielsweise Silber enthält. Allerdings wird der Fachmann in dieser Schrift dazu angeleitet, eine wasserfreie oder wasserbindende Salbe zu verwenden, um eine zu schnelle und zu hohe Freisetzung des antibakteriellen Metalls zu vermeiden. Hierdurch eignet sich die Wundauflage nicht für die Behandlung nekrotischer Wunden oder von Wunden, die teilweise von Nekrosen durchsetzt sind. Zudem ist die antibakterielle Wirkung solcher Wundauflagen unzureichend bei Wunden, die Biofilme aufweisen.

Folglich besteht ein Bedarf nach einem Mittel zur Wundabdeckung, das ausreichende antimikrobielle Wirksamkeit gegenüber Biofilmen zeigt, die Wunde befeuchtet, chronische Wunden in ihrer Heilung fördert, und zudem atraumatisch und schmerzfrei entfernt werden kann. Um dem klinischen Alltag gerecht zu werden, sollte das Mittel der Wahl zudem unmittelbar einsetzbar, bei längerer Lagerung über eine ausreichende Stabilität verfügen und beständig gegenüber möglichen Temperaturschwankungen sein.

### Zusammenfassung der Erfindung

Die vorangestellte Aufgabe wird gelöst durch Bereitstellung einer Wundkontaktschicht umfassend ein netzförmiges Substrat, welches mit einer Emulsion teilweise oder vollständig beschichtet ist, wobei die Emulsion eine antimikrobiellen Zusammensetzung enthaltend
a) ionisches Silber und / oder Silbernitrat,
b) ionisches Zink und / oder Zinknitrat und / oder Zinksulfat, und
c) EDTA
umfasst.

Die erfindungsgemäße Wundkontaktschicht hat hervorragende atraumatische Eigenschaften, d.h. sie verbindet sich nicht mit der Wunde oder Wundbestandteilen. Weder kann Gewebe in die Wundkontaktschicht einwachsen, noch kann die Wundkontaktschicht am Wundbett ankleben. Durch die enthaltene Emulsion wird der Wundverschluss angeregt und die Wundränder vor einem Aufweichen (Mazeration) geschützt. Schließlich ist die enthaltene antimikrobielle Zusammensetzung nicht nur gegen humanpathogene Bakterien, sondern auch gegen Biofilme wirksam. Zudem kann die Wundkontaktschicht nach dem Anbringen auf dem Patienten durch einen passenden Sekundärverband überlagert und fixiert werden. Die Art des Sekundärverbands kann dabei je nach Wundart spezifisch ausgewählt werden, um die bestmögliche Versorgung zu gewährleisten.

Die erfindungsgemäße Wundkontaktschicht kann auch dann zum Einsatz kommen, wenn sich in der zu behandelnden Wunde antibiotikaresistente Bakterien befinden. Während Antibiotika üblicherweise organische Verbindungen sind, die von resistenten Bakterien beispielsweise abgebaut werden, ist ein derartiges Auftreten von Resistenzen bei der antimikrobiellen Zusammensetzung der vorliegenden Erfindung nicht zu erwarten.

Die erfindungsgemäße Wundkontaktschicht kann, auch bei nicht infizierten Wunden, wundheilungsfördernde Eigenschaften aufweisen.

Nachfolgend wird erläutert, wie die Wundkontaktschicht strukturell und chemisch beschaffen sein kann, um in der Praxis den größtmöglichen Nutzen zu erbringen.

### Detaillierte Beschreibung der Erfindung

Der Begriff "medizinisch akzeptables Material" im Sinne der Erfindung ist eine ungiftige, fusselfreie und stabile Substanz, die sich unter Normalbedingungen weder in polaren noch in unpolaren Verbindungen löst und weder von den Absonderungen tierischer noch bakterieller Zellen in nennenswertem Ausmaß abgebaut oder verflüssigt werden kann.

Der Begriff "stabile Emulsion" meint, dass sich die polaren und die unpolaren Bestandteile einer Emulsion (z.B. Wasser und Öl) bei einer Temperaturbereich von 20°C bis 25°C und einem Druck von einem bar nicht in zwei Phasen trennen. Wenn unter diesen Bedingungen innerhalb eines Zeitraums von sieben Tagen keine Entmischung einsetzt, kann davon ausgegangen werden, dass die Emulsion stabil ist.

Mit "t-EDTA" ist die Verbindung Tetra-Natrium-Ethylendiamintetraessigsäure gemeint.

Der Begriff "Biofilm" meint einen dünnen, flächig ausgebreiteten Schleimfilm, in dem Populationen von Mikroorganismen vorliegen. Der Schleimfilm wird von den Mikroorganismen gebildet und ist eine die Mikroorganismen einschließende Matrix aus extrazellulärem polymerem Material. Typischerweise handelt es sich bei den Populationen von Mikroorganismen um Mischpopulationen. Biofilme bilden sich auf Oberflächen aus, zu denen auch Wundgewebe gehören kann. Die im Biofilm organisierten Mikroorganismen zeigen eine erhöhte Widerstandskraft gegen herkömmliche Antibiotika, Desinfektionsmittel und gegenüber dem Immunsystem höher entwickelter Lebewesen.

Der Begriff "Biofilm reduzierend" umfasst sowohl die Abtötung von Bakterien im Biofilm (desinfizierende Eigenschaften) als auch das Aufbrechen von chemischen Bindungen und den Entzug chemischer Bindungspartner aus der Biofilm bildenden extrazellulären Matrix. Letzteres setzt die Abwehrkraft der zuvor im Biofilm organisierten Mikroorganismen gegenüber Einflüssen wie Desinfektionsmitteln, Antibiotika, dem Immunsystem oder Umwelteinflüssen allgemein herab. Als Biofilm reduzierend werden im Rahmen der vorliegenden Erfindung nur derartige Substanzen verstanden, die geeignet sind, auf offene Wunden aufgebracht zu werden, ohne eine nennenswerte Schadwirkung am zu behandelnden Individuum zu entfalten.

Der Begriff "netzförmiges Substrat" meint, dass das Substrat in Form eines Netzes mit Stegen und zwischen den Stegen liegenden Öffnungen vorliegt. Insbesondere sind Öffnungen vorhanden, die sich von einer Seite des netzförmigen Substrates zur gegenüberliegenden Seite erstrecken. Unter einer Öffnung im Sinne der vorliegenden Erfindung ist somit ein durch das Substrat hindurchgehender Hohlraum zu verstehen, der geeignet ist, eine Flüssigkeit wie zum Beispiel Wundexsudat von einer Oberfläche des Substrats zur gegenüberliegenden Oberfläche des Substrats passieren zu lassen.

Eine netzförmige Struktur im Sinne der vorliegenden Erfindung kann beispielsweise durch ein Gitter, ein Gewebe, ein Gewirke, ein perforiertes Vlies oder durch eine perforierte Folienlage erzeugt werden.

Der Ausdruck "Kolonie bildende Einheit" (CFU) meint eine einzelne teilungsfähige Zelle eines Einzellers, insbesondere eines humanpathogenen Einzellers oder Bakteriums.

"Beschichtet" meint, dass eine Oberfläche eines Festkörpers mittels einer Substanz, die sich von der Struktur des Festkörpers unterscheidet, zumindest teilweise bedeckt bzw. überlagert wird.

Der Ausdruck "konfiguriert zum Auflegen auf eine Wunde" meint, zum Auflegen auf eine Wunde zu einem therapeutischen Zweck im Sinne einer Wundbehandlung vorgesehen und geeignet.

Der Ausdruck "atraumatisch" meint, dass ein Wundversorgungsprodukt sich nicht mit der Wunde fest verbindet, also beispielsweise nicht in der Wunde eintrocknet oder mit dieser verwächst und dass eine schmerzfreie Entfernung des Produktes ohne Störung des Heilungsprozesses möglich ist.

Die vorliegende Erfindung betrifft eine Wundkontaktschicht. Diese Wundkontaktschicht kann allein verwendet werden oder nach dem Anbringen auf eine Wunde bei Bedarf durch einen absorbierenden Sekundärverband überlagert werden oder mittels Sekundärverband, Klebefolie, Klebestreifen oder anderer Fixiermittel am Ort der Wunde befestigt werden. Bei Bedarf, z.B. wenn der Sekundärverband mit Wundexsudat gesättigt ist oder wenn sich die angebrachte Klebebefestigung löst, können Sekundärverband oder Klebemittel ersetzt werden, ohne die Wundkontaktschicht von der Wunde lösen zu müssen. Die Wundruhe bleibt auf diese Weise erhalten und ein unerwünschtes Eintrocknen des Sekundärverbands in der Wunde wird vermieden.

Vorteilhaft ist, dass die Wundkontaktschicht mittels der enthaltenen Emulsion an der Haut atraumatisch anhaftet. Ein zusätzlicher Sekundärverband bietet den Vorteil, dass ein Verrutschen der Wundkontaktschicht verhindert wird. In den meisten Fällen muss die Wundkontaktschicht nicht bis zur Applikation eines Fixiermittels an der Wundstelle festgehalten werden, sondern hält zunächst von alleine, so dass dem Anwender beide Hände für die Vorbereitung des Sekundärverbands zur Verfügung stehen.

Die erfindungsgemäße Wundkontaktschicht eignet sich für akute und blutende Wunden, chronische Wunden, nässende Wunden, Brandwunden (bis zu Verbrennungen 2. Grades), Wunden, die nekrotisches Gewebe oder Fibrinbeläge enthalten, vor allem für infizierte Wunden und darunter insbesondere biofilmhaltige Wunden. In der Praxis treten häufig Mischformen dieser Wunden auf, z.B. infizierte, teilnekrotische, chronische Wunden mit Biofilm. In solchen Fällen zeigen sich die vorteilhaften Eigenschaften der erfindungsgemäßen Wundkontaktschicht besonders deutlich, da mit nur einem Produkt eine Vielzahl unterschiedlicher pathologischer Wundmilieus behandelt wird.

Die Wundkontaktschicht umfasst mindestens ein netzförmiges Substrat und eine Emulsion, welche eine antimikrobielle Zusammensetzung enthält. Weitere Bestandteile, wie beispielsweise ein umlaufender Kleberand können bei Bedarf hinzugefügt werden, um die Wundkontaktschicht an den vorgesehenen Einsatzzweck anzupassen. Dies wird an anderer Stelle näher erläutert.

Im Rahmen der vorliegenden Erfindung kann vorgesehen sein, dass sich die Emulsion ausschließlich auf der ersten Seite des Substrats befindet und damit auf derjenigen Seite, welche der Wunde zugewandt ist. Auf diese Weise ist die für den Wundkontakt vorgesehene Seite eindeutig vom Anwender erkennbar und bedarf keiner weiteren Kennzeichnung. Außerdem hat diese Ausführung den ökonomischen Vorteil, dass Emulsionsmaterial gespart wird, ohne dass die Versorgung der Wunde darunter leidet.

Das in der erfindungsgemäßen Wundkontaktschicht enthaltene Substrat ist ein flexibler und elastischer Festkörper, der sich der Form der Körper- oder Wundoberfläche anpassen kann. Das Substrat besteht aus einem festen, unlöslichen, medizinisch akzeptablen Material oder aus daraus hergestellten Fasern, wobei das Material vorzugsweise über eine Kristallgitterstruktur verfügt und nicht amorph ist. Bei dem Material kann es sich um ein Polymer- oder um eine Polymermischung handeln. Bevorzugt ist das Substrat flach bzw. planar, so dass es eine im Wesentlichen einheitliche Höhe aufweist und weder die erste noch die zweite Seite Erhebungen aufweisen. Geringfügige produktionsbedingte Toleranzen sind hierbei zu vernachlässigen, so dass im Sinne der Erfindung eine einheitliche Höhe auch bei Abweichungen von +/- 5 % als gegeben gelten soll. Ein flach ausgebildetes Substrat kann in der Aufsicht eine rechteckige, quadratische, ovale oder runde Form haben, wobei ovale oder runde Formen sich besonders für Wunden an Gelenken eignen und rechteckige oder quadratische Formen sich einfacher verpacken lassen und den Lagerplatz effizienter nutzen. Die Wundkontaktschicht kann die Form des Substrats haben, was jedoch nicht zwingend erforderlich ist.

Das Substrat hat eine erste Seite, welche in Benutzung der Wunde zugewandt ist (proximale Ausrichtung) und eine der ersten Seite gegenüberliegende zweite Seite, welche in Benutzung von der Wunde abgewandt ist (distale Ausrichtung).

Das Substrat ist netzförmig ausgestaltet. Die netzförmige Ausgestaltung erlaubt den Durchtritt von Wundexsudat ausgehend von der ersten Seite des Substrats in Richtung der zweiten Seite des Substrats. Daneben verleiht die Netzform dem Substrat eine ausgesprochene Flexibilität, da die Materialspannung beim Auflegen auf eine Wunde und der damit einhergehenden (reversiblen) Verformung minimiert wird. Zu guter Letzt wird durch das Netz die Kontaktfläche zur Wunde und damit die Grenzflächenspannung reduziert, so dass sich das Substrat besonders schonend von der Wunde ablösen lässt.

Eine mögliche Netzform ist die Form eines Gitters, bei welchem das Substrat Stege in Form von Längs- und Querstreben enthält, welche im Wesentlichen rechtwinklig zueinander angeordnet sind, so dass die Öffnungen im Substrat die Form von Rechtecken einnehmen. Gemäß einer bevorzugten Ausführungsform liegt die netzförmige Ausprägung in Form eines gleichförmigen Musters vor, so dass alle Öffnungen oder Poren im Wesentlichen dieselbe räumliche Ausdehnung und denselben Abstand zueinander haben. Der hieraus resultierende Vorteil ist, dass über die gesamte Fläche der ersten und zweiten Seite im Wesentlichen gleichbleibende Parameter und Kräfte vorherrschen und die antimikrobiellen Zusammensetzung folglich gleichmäßig an allen Positionen einer Wunde wirken kann.

Bevorzugt enthält das Substrat Fasern. Das Substrat kann ausschließlich aus Fasern bestehen. Diese Fasern können eines oder mehrere der folgenden Materialien enthalten oder daraus bestehen: Polyamid, Polyester, Polyacryl, Polyurethan, Polypropylen, Polyvinylalkohol, Baumwolle, Viskose und Mischungen daraus. Ein Beispiel für eine mögliche Mischung von Fasern unterschiedlicher Materialien ist eine Kombination von Polyester mit Baumwolle oder von Polyester mit Viskose. Bei Verwendung von Polyamid als Fasermaterial kann das Polyamid in Form von Nylon vorliegen.

Sämtliche der aufgeführten Fasern können zur Verwendung im netzförmigen Substrat als Gewirke ausgestaltet sein. Auch ist es möglich die Fasern als Tüll anzuordnen. Ein Netz in Form eines Tülls kann durch eine repetitive Anordnung von Sechsecken (hexagonale Anordnung innerhalb des Tülls) oder Rauten (Rautenanordnung innerhalb des Tülls) charakterisiert sein.

Alle oben aufgeführten Fasern bzw. Faserarten haben als netzförmiges Substrat vorliegend zum einen strukturgebende Eigenschaften und darüber hinaus die vorteilhafte Eigenschaft Flüssigkeiten wie Wundexsudat an einen optionalen Sekundärverband zu transferieren, wo sie gespeichert und später entfernt werden können. In Kombination mit einer wasserhaltigen Emulsion wird zudem die Wunde in geringem Umfang gespült und dabei die Ablösung ggf. vorhandener Nekrosen gefördert. Im Allgemeinen sind Fasern natürlichen Ursprungs dabei hydrophiler als synthetische Fasern. Dafür übertreffen synthetische Fasern die Naturfasern hinsichtlich ihrer Bindungsaffinität gegenüber unpolaren Substanzen wie Ölen, Fetten und Wachsen. Die synthetischen Fasern haben aus diesem Grund hervorragende Bindeeigenschaften gegenüber W/O-Emulsionen, mit welchen die erfindungsgemäße Wundkontaktschicht beschichtet werden kann. Zudem sind sie preiswert, reißfest, verfügen über eine hohe Zugfestigkeit und lassen sich einfach verarbeiten. Durch ihren unpolaren Charakter zeigen sie kein Rückhalteverhalten gegenüber den Kationen der antimikrobiellen Zusammensetzung, was in deren optimaler Freisetzung resultiert.

Naturfasern haben bieten den Vorteil einer besseren Ökobilanz und bilden besonders stabile Beschichtungen mit O/W-Emulsionen aus. Bei Einsatz von Naturfasern kann es jedoch notwendig sein, die Konzentration antimikrobiellen Zusammensetzung zu erhöhen oder mit eine größeren Menge an Emulsion zu beschichten, um die gleiche Wirksamkeit wie bei synthetischen Fasern zu erhalten, da Naturfasern aufgrund ihrer polaren Eigenschaften mit diesen geladenen Teilchen interagieren und deren Freisetzung in geringem Maße hemmen können.

Daneben ist es auch möglich, dass das netzförmige Substrat nicht aus Fasern, sondern aus einer insbesondere homogenen Masse besteht. Die Masse sollte elastisch, biegsam, beständig und natürlich biokompatibel sein. Eine geeignete Masse ist Silikon, weswegen im Rahmen der Erfindung das netzförmige Substrat aus Silikon bestehen oder solches enthalten kann. Silikon bietet den Vorteil einer größtmöglichen Biokompatibilität. Es ist hypoallergen und weist keinerlei Toxizität auf. Darüber hinaus ist es auch biochemisch innert. Es kann nicht von Mikroorganismen bzw. Krankheitserregern zersetzt oder als Nährsubstrat genutzt werden, verbindet sich nicht mit dem Wundgewebe und kann schmerzfrei abgelöst werden. Aus diesen Gründen wird es gerne im medizinischen Bereich verwendet. Dennoch hat Silikon einen Nachteil. Es ist bekannt für seine okklusive Eigenschaft, die die Hautatmung behindert und die Absorption von Wundexsudat in Wundauflagen hemmt. Da der Träger der erfindungsgemäßen Wundkontaktschicht jedoch durch seine netzförmige Struktur über Öffnungen verfügt, bleibt der gewünschte Stoffaustausch erhalten.

Zwar ist es im Rahmen der Erfindung grundsätzlich möglich eine Folie als netzförmiges Substrat auszubilden. Bei trockenen oder durch Schorf bedeckten Wunden können folienhaltige netzförmige Substrate eine Alternative zu anderen Substratformen darstellen, wenngleich die Emulsion bei einer Folie (im Gegensatz zu einem faserbasierten Substrat) nur auf den Oberflächen der Folien aufgetragen werden kann und nicht zusätzlich im Inneren des Substrats. Vorzugsweise ist das von der Wundkontaktschicht umfasste netzförmige Substrat daher keine Folie und enthält auch keine Folie.

Im Rahmen der Erfindung kann das Substrat bei Bedarf frei sein von bestimmten Substanzen. Beispielsweise kann das Substrat frei sein von Gelatine und Kollagen oder generell frei sein von Substanzen tierischen Ursprungs oder von Substanzen natürlichen Ursprungs. Substrate, die frei sind von derartigen Stoffen, sind in aller Regel resistent gegenüber unerwünschtem biologischen Abbau - z.B. beim Behandeln einer infizierten Wunde.

Gemäß einer Ausführungsform ist das netzförmige Substrat mit elementarem, also nicht ionischem Silber beschichtet. Dieses zusätzliche Silber, welches zusätzlich zu dem in der antimikrobiellen Zusammensetzung vorhandenen ionischen Silber vorhanden ist, dient als zusätzliches Reservoir. Da es nicht in der Emulsion vorhanden ist, sondern von dieser überdeckt wird, ermöglicht es eine besonders langfristige Abgabe von Silber an die Wundstelle. Hierbei gilt, je mehr Emulsion auf das Substrat beschichtet wurde und je hydrophober die Emulsion, desto zeitverzögerter wird das zusätzliche Silber aus dem Substrat abgegeben.

Bedingt durch den netzförmigen Aufbau sind im Substrat Öffnungen enthalten, wobei Abstände zwischen einzelnen Fasern bzw. Faserfilamenten wie sie in jedem Textilverbund (Gewirke, Tüll etc.) vorkommen, nicht als Öffnungen im Sinne der Erfindung gelten.

Die im netzförmigen Substrat vorhandenen Öffnungen können beliebig geformt sein. Möglich ist eine kreisförmige oder elliptische Ausgestaltung, da auf diese Weise Ecken vermieden werden, welche sich nach längerer Tragedauer auf einer Wunde für den Patienten unangenehm anfühlen können. Alternativ ist eine quadratische oder trigonale Öffnungsfläche ebenfalls möglich. Die Größe der Öffnungen kann im Rahmen der vorliegenden Erfindung variieren, wobei diese groß genug ausfallen sollte, um den Abfluss von Exsudat zu ermöglichen bzw. nicht zu verlangsamen. Die Öffnungen können eine Fläche von mindestens 0,01 mm² haben und bevorzugt eine Fläche von mindestens 0,1 mm². Poren sind keine Öffnungen im Sinne der Erfindung. Besonders gut geeignet sind Öffnungen mit einer Projektionsfläche von mindestens 0,1 mm², entsprechend einem Kreisdurchmesser von 0,357 mm. Andererseits besteht bei zu großen Öffnungen die Gefahr einer Verklebung mit der Wunde und dem Einwachsen von Granulationsgewebe in die Öffnungen. Gut geeignet sind Öffnungen mit einer Größe von 0,1 mm² bis 10 mm², von 0,3 mm² bis 8 mm², besonders 0,5 bis 5 mm². Es ist auch möglich Öffnungen zu nutzen, die eine Öffnungsfläche von maximal 1 mm² haben.

Das Substrat weist eine Vielzahl von Öffnungen und einen geschlossenen Bereich (Stege) auf, der die Öffnungen umgibt. Das Verhältnis der Gesamtfläche der Öffnungen zu der geschlossenen Fläche des Substrats beträgt 1:1,5 bis 1:4, bevorzugt 1:2 bis 1:3.

Im Rahmen der vorliegenden Erfindung kann eine Emulsion eine Salbe, eine hydrophobe Salbe, eine hydrophile Salbe, eine wasseraufnehmende Salbe, eine Öl-in-Wasser-Emulsion (O/W-Emulsion), eine Wasser-in-Öl-Emulsion (W/O-Emulsion), ein Gel oder eine Paste sein. Die Emulsion kann wasserbindend, fettbindend oder beides sein. Sie enthält mindestens einen polaren und mindestens einen unpolaren Bestandteil.

Der polare Bestandteil kann Wasser oder eine andere ungiftige polare Substanz sein, die bevorzugt bei Raumtemperatur als Flüssigkeit vorliegt. Ein mögliches Beispiel ist PEG, welches als Flüssigkeit (Molekülmasse von unter 600 g/mol) oder als Pulver (Molekülmasse ab 2000 g/mol) oder als Paste (Molekülmasse zwischen 600 und 2000 g/mol) eingesetzt werden kann und in der fertigen Emulsion gelöst vorliegt.

Der unpolare Bestandteil kann beispielsweise eine Substanz sein, die zu den Ölen, Fetten, Fettsäuren, Wachsen, Triglyceriden sowie den Kohlenwasserstoffen zählt. Bei den Kohlenwasserstoffen sind die aliphatischen Kohlenwasserstoffe bevorzugt. Besonders bevorzugt sind gesättigte, aliphatische Kohlenwasserstoffe. Der Einsatz von ungesättigten Kohlenwasserstoffen ist jedoch möglich, so lange die resultierende Emulsion eine ausreichende Viskosität zeigt, um aus ihr eine stabile Beschichtung auf dem Substrat zu erzeugen. Diese unpolaren Substanzen reduzieren die Freisetzung der Komponenten aus der antimikrobiellen Zusammensetzung (Barriereeffekt). Dies kann gewünscht sein, wenn die Wundkontaktschicht längere Zeit (z.B. mehrere Tage) auf der Wunde verbleiben und dabei kontinuierlich die antimikrobiellen Komponenten freisetzen soll.

Daneben sind Mischungen mehrerer polarer und unpolarer Substanzen als Bestandteil der Emulsion möglich.

Die Emulsion kann darüber hinaus ein oder mehrere Emulgatoren sowie wasserbindende Bestandteile enthalten. Beispiele für wasserbindende Bestandteile sind Propylenglykol, Ethylenglykol, Polyethylenglykol (PEG), insbesondere PEG 2000 (Molekulargewicht 2 kDa) und Mischungen aus mindestens zwei dieser Bestandteile. Diese oder andere wasserbindende Bestandteile können beispielsweise 1 bis 20 Gew.-% der Emulsion ausmachen. Empfohlen wird ein Anteil von 5 bis 15 Gew.-%, besonders empfohlen 8 bis 12 Gew.-%. Alternativ kann der Anteil auch mindesten 5 Gew.-% betragen. Die wasserbindenden Bestandteile sind auch zur Bindung anderer polarer Flüssigkeiten als Wasser geeignet. Beispiele für andere polare Flüssigkeiten sind Ethanol und Glycerin. Die wasserbindenden Bestandteile steuern die Freisetzung der antimikrobiellen Zusammensetzung. Je höher ihr Anteil und der Anteil der polaren Flüssigkeit an der Emulsion, desto mehr Komponenten der antimikrobiellen Zusammensetzung werden innerhalb eines bestimmten Zeitraums an die Umgebung, insbesondere an das Gewebe, abgegeben. Beispiele für Emulgatoren sind Stearate, Cetearyl Glucoside, Lecithin und Mischungen aus mindestens zwei dieser Bestandteile. Diese und andere Emulgatoren können beispielsweise einen Anteil von 0,5 bis 5 Gew.-% in der Emulsion ausmachen.

Unter einer hydrophoben Salbe wird eine Salbe verstanden, deren Grundlage keine polaren Bestandteile oder Emulgatoren enthält und deshalb nicht in der Lage ist, Wasser aktiv zu binden. Eine hydrophobe Salbe kann als Grundlage eine Mischung höherer aliphatischer Alkane enthalten. Beispielsweise kann Vaseline eingesetzt werden. Im näheren Sinn wird unter Vaselin eine hochviskose Substanz verstanden, die sowohl verzweigtkettige feste Kohlenwasserstoffe als auch geradkettige flüssige Kohlenwasserstoffe enthält. Eine bevorzugte Variante von Vaselin ist das sog. weiße Vaselin *(Vaselinum album),* welches aufgrund seiner Reinheit eine besonders hohe Qualität hat. Zu den Vorteilen von Vaseline zählen eine gute Biokompatibilität und hervorragende Hautverträglichkeit. Durch die okklusiven Eigenschaften und die kaum vorhandene Hautaufnahme ist Vaseline gut geeignet, um längerfristigen Schutz bei Defekten der Hautbarriere zu bieten. Da Vaseline von Mikroorganismen nahezu gar nicht metabolisiert werden kann, können Krankheitserreger es nicht als Kohlenstoffquelle nutzen. Alternativ oder zusätzlich kann eine hydrophobe Salbe Silikonöl enthalten. Beispielsweise kann das Silikonöl in einer Konzentration von 3 bis 50 Gew.-% oder 5 bis 30 Gew.-% in der Salbe enthalten sein.

Unter einer wasseraufnehmenden Salbe wird eine Salbe verstanden, die neben lipophilen Grundstoffen auch amphiphile Hilfsstoffe enthält und somit zur Wasseraufnahme fähig ist. Amphiphile Hilfsstoffe wirken hierbei als Emulgatoren. Für wasseraufnehmende Salben sind W/O-Emulgatoren geeignet, bevorzugt Partialester aus mit verschiedenen Fettsäuren verestertem Glycerin oder Diglycerin, besonders bevorzugt Diglycerinpartialester mit verschiedenen mittel- und langkettigen Fettsäuren. Ein Beispiel für einen geeigneten Emulgator ist Cetylstearylalkohol. Wasseraufnehmende Salben können Lanolin (Wollwachs) enthalten. Beispielsweise kann Lanolin in einer Konzentration von 3 bis 50 Gew.-% oder von 10 bis 30 Gew.-% enthalten sein. Eine bevorzugte wasseraufnehmende Salbe enthält 40 bis 70 Gew.-% Triglyceride, 15 bis 30 Gew.-% Diglycerinpartialester, 2 bis 15 Gew.-% Polyethylenglykol (PEG) und 5 bis 25 Gew.-% an Wasser oder einer anderen polaren Flüssigkeit. Bevorzugt enthält die wasseraufnehmende Salbe 5 bis 15 Gew.-% PEG, besonders bevorzugt 5 bis 12 Gew.-% PEG.

Unter einer hydrophilen Salbe wird eine Salbe verstanden, die mit Wasser mischbar ist. Eine hydrophile Salbe enthält typischerweise Polyethylenglykole. Hydrophile Salben unterstützen den Durchtritt von Wundexsudat durch die Wundkontaktschicht und gleichzeitig auch die Diffusion und Freisetzung der Ionen aus der antibakteriellen Zusammensetzung.

Unter einer Emulsion wird eine Salbe verstanden, die neben einer Lipidphase eine wässrige Phase enthält. Sowohl Emulsionen vom W/O-Typ als auch Emulsionen vom O/W-Typ können im Rahmen der Erfindung eingesetzt werden. Es sind auch Verteilungen möglich, bei denen zwei oder mehrere angrenzende Phasen ausgebildet werden.

Unter einem Gel wird eine Zubereitung verstanden, bei denen Flüssigkeiten durch Gelgerüstbildner verfestigt werden. Für die Wundversorgung bevorzugte Gele sind hydrophile wasserhaltige Gele, die auch als Hydrogele bezeichnet werden. Geeignete Gele enthalten beispielsweise neben Wasser (sog. Hydrogele) oder einer anderen polaren biokompatiblen Flüssigkeit mindestens einen Gerüstbildner bzw. Gelbildner wie z.B. modifizierte oder nicht-modifizierte Polysaccharide, insbesondere Hydroxymethylcellulose, Carboxymethylcellulose, Stärke, modifizierte Stärke, Chitosan, Alginat oder Agar, sowie Gelatine, Kollagen, Polyvinylalkohol, Polyglycidol, oder Polyacrylamid. Diese Bestandteile oder eine Mischung daraus können 0,5 bis 30 Gew.-% der Emulsion ausmachen. Im Rahmen der Erfindung enthält ein Gel neben einer polaren Flüssigkeit immer ein oder mehrere unpolare Bestandteile, wobei der Anteil der polaren Flüssigkeit im Gel mindestens 50 Gew.-%. beträgt. Gele eignen sich besonders gut zur Behandlung von Wunden, in denen Fibrinbeläge oder Nekrosen vorhanden sind.

Unter einer Paste wird eine Salbe mit einem Feststoffanteil verstanden, wobei eine Festkörperdispersion vorliegt. Eine Salbe kann beispielsweise einen Feststoffanteil von 0,1 bis 15 Gew.-% enthalten. Bevorzugt beträgt der Feststoffanteil in der Salbe 0,3 bis 5 Gew.-% und besonders bevorzugt 0,5 bis 3 Gew.-%. Die antibakterielle Zusammensetzung - sofern partiell ungelöst und als Feststoff vorliegend - ist bei den angegebenen Feststoffanteilen unberücksichtigt. Der optimale Feststoffanteil ist abhängig von der Art des Feststoffes oder der Feststoffe und ob diese z.B. als Wirkstoff oder lediglich als Hautpflegebestandteil enthalten sind. Eine Paste stellt in der Regel eine feststoffstabilisierte Salbe dar. Voraussetzung ist, dass die Salbe wasserhaltig ist und dass zumindest ein Teil der enthaltenen Feststoffpartikel eine Hydrathülle ausbilden können, wie dies beispielsweise bei anorganischen Ionen der Fall ist.

Darüber hinaus kann die Emulsion auch als Fluid vorliegen. Eine Emulsion, die die antimikrobielle Zusammensetzung enthält, kann darüber hinaus als disperses Fluid vorliegen.

Um die Konsistenz der Emulsion (z.B. wenn diese als Salbe vorliegt) einzustellen, können die folgenden Bestandteile beigemengt werden. Jeder dieser Bestandteile kann in einer Konzentration von 1 bis 10 Gew.-% oder 2 bis 9 Gew.-% oder aber 3 bis 8 Gew.-% vorhanden sein: Cetylalkohol, Stearylalkohol, Isopropylmyristat. Es wird darauf hingewiesen, dass unpolare Bestandteile nach dem Auflegen der Wundkontaktschicht auf das Gewebe durch die Körperwärme an Viskosität verlieren, was durchaus im Sinne der Erfindung ist und die Verteilung der Emulsion in Wundbereich fördert, so dass ein direkter Kontakt zwischen Substrat und Wundbett nicht notwendig ist, um die antimikrobielle Zusammensetzung in das Wundbett einzubringen und damit einhergehend auch eine vorteilhafte Tiefenwirkung zu erzielen.

Schließlich kann vorgesehen sein, dass die Emulsion mindestens einen Bestandteil ausgewählt aus der Gruppe der Mono-, Di- oder Triglyceride, Fettsäureester, Oligomere des Glycerin, Fettalkohole, Fettsäureester, ethoxylierten Fettalkohole, ethoxylierten Fettsäuren, Polyethoxylen-Derivate oder dimerisierten Fettsäureester oder Gemische der genannten Substanzen umfasst, wobei diese beispielsweise in einem Gehalt von 30 bis 70 Gew.-% oder 40 bis 70 Gew.-% in der Emulsion enthalten sein können. Unter diesen genannten Substanzen werden die folgenden bevorzugt: Di- und Triglyceride der Caprylsäure und / oder der Caprinsäure und / oder der Isostearinsäure und / oder der (Iso-)Stearinsäure und / oder der 12-Hydroxystearinsäure sowie Lanolin. Insbesondere kann die Emulsion ein Gemisch aus verschiedenen Mono-, Di- und Triglyceriden und/oder Fettsäureester, Oligomere des Glycerins und/oder Polyethylenglykolen umfassen. Vorteilhaft ist eine Zubereitung, die 1 bis 70 Gew.-% an Mono-, Di- und Triglyceriden, 1 bis 70 Gew.-% Fettsäureester Oligomere des Glycerins, 1 bis 20 Gew.-% Polyethylenglykol und 1 bis 10 Gew.-% von Wasser und antimikrobieller Zusammensetzung umfasst.

Weiterhin kann die Emulsion bzw. die oben beschriebenen Emulsionszusammensetzungen auch einen Konservierungsstoff oder einen Stabilisator in einer Menge von 0,1 bis 2 Gew.-% enthalten. Beispiele für geeignete Konservierungsstoffe sind beispielsweise Benzoesäure, Sorbinsäure oder Parabene. Beispiele für geeignete Stabilisatoren sind Ascorbylpalmitate und Tocopherol. Da die emulsionshaltige Wundkontaktschicht in der Regel vor der Verwendung sterilisiert wird, kann in den meisten Fällen auf die Verwendung von Konservierungsstoffen verzichtet werden, um den Produktionsaufwand gering zu halten. Vorzugsweise kann auch vorgesehen sein, dass die Emulsion generell keine Konservierungsstoffe und / oder Stabilisatoren enthält, um die Wahrscheinlichkeit für das Auftreten von Allergien und Unverträglichkeitsreaktionen weitestgehend zu reduzieren.

Der Gehalt an polaren Flüssigkeiten, insbesondere an Wasser, in der Emulsion kann 1 bis 50 Gew.-% betragen. In manchen Fällen (z.B. wenn die Emulsion als Gel vorliegt) können auch mehr als 50 Gew.-% an polaren Flüssigkeiten gewünscht und sinnvoll sein. Bevorzugt enthält die Emulsion 15 bis 45 Gew.-% polare Flüssigkeiten, besser 20 bis 40 Gew.-% polare Flüssigkeiten und am besten enthält die Emulsion 25 bis 35 Gew.-% polare Flüssigkeiten, da eine solche Konzentration eine besonders gute Freisetzung der antimikrobiellen Zusammensetzung ermöglicht und gleichzeitig eine hervorragende Stabilität der Emulsion gewährleistet. Zudem hat sich dieser Bereich als besonders Wirkungsvoll gegenüber dem problematischen Erreger S. *aureus* erwiesen. Es können zwei oder mehr verschiedene polare Flüssigkeiten in der Emulsion vorhanden sein. Beispiele für mögliche Kombination aus polaren Flüssigkeiten sind Wasser und Ethanol oder Wasser und Glycerin.

Bevorzugt handelt es sich bei der Emulsion um eine stabile Emulsion. Überraschenderweise hat sich gezeigt, dass die erfindungsgemäße antimikrobielle Zusammensetzung einen stabilisierenden Effekt auf die Emulsion hat, so dass eine feststoffstabilisierte Emulsion entsteht. Dies geht mit vorteilhaften Wirkungen einher. Neben einer Stabilisierung des pH-Wertes (auch in Abwesenheit möglicher Puffersysteme) bietet die feststoffstabilisierte Emulsion eine hervorragende Langzeithaltbarkeit, ohne dass es zu einer Trennung der wässrigen und der öligen Phase kommt. Diese Langzeitstabilität ist bei medizinischen Artikels eine besonders gewünschte Eigenschaft, da derartige Produkte von medizinischen Einrichtungen im Rahmen rabattierter Großbestellungen erworben werden und bis zu ihrem Einsatz (der im Regelfall nicht absehbar ist) gelagert werden müssen. Während der Lagerung können die Produkte durchaus saisonal bedingten Temperaturschwankungen unterliegen. Die erfindungsgemäße feststoffstabilisierte Emulsion bietet verbesserte Beständigkeit sowohl bei verlängertem Lagerungszeitraum als auch gegenüber Temperaturschwankungen. Dies ist auch dem Sterilisationsprozess zuträglich.

Unterschiedliche Formulierungen der Emulsion können genutzt werden, um die für den Einsatzzweck gewünschten Eigenschaften zu vermitteln. So kann beispielsweise die Zugabe von Triglyceriden genutzt werden, um eine Rückfettung zu ermöglichen und die antimikrobielle Tiefenwirkung zu steigern. Ein hoher Anteil von polaren Flüssigkeiten kann das Ablösen von Fibrin und Nekrosen aus der Wunde fördern. Ziel bei der Wahl der Inhaltsstoffe und deren Konzentration in der Emulsion sollte ein Wundverschluss sein, ohne dass Krankheitserreger im Gewebe zurückbleiben.

Die "antimikrobielle Zusammensetzung" kann im Rahmen der vorliegenden Erfindung insbesondere in folgenden Zuständen vorliegen:
a) als Mischung aus ionischem Silber und / oder Silbernitrat sowie ionischem Zink und / oder Zinknitrat und / oder Zinksulfat sowie EDTA oder Tetra-Natrium EDTA,
b) als Komplexverbindung Ag₂Zn(EDTA),
c) als Lösung der besagten Komplexverbindung in einer polaren Flüssigkeit wie Wasser oder einem anderen ungiftigen, polaren Lösungsmittel, oder
d) als Lösung der Ausgangskomponenten Silbernitrat, Zinksulfat bzw. Zinksulfatmonohydrat und Tetrasodium EDTA in einer polaren Flüssigkeit wie Wasser oder einem anderen ungiftigen, polaren Lösungsmittel, so dass die besagte Komplexverbindung ausgebildet wird.

Sofern nicht anders ausgeführt, liegt die "antimikrobielle Zusammensetzung" primär in Form der unter a) genannte Mischung vor und zwar bevorzugt in den ionischen Formen. Allerdings können auch andere Salze von Silber und Zink als deren Nitrate eingesetzt werden, solange eine Ausbildung der unter b) angegeben Komplexverbindung durch die Reaktanden möglich ist. Dabei müssen nicht sämtliche Kationen von Silber und Zink zum Komplex abreagieren. Die Ausbildung eines chemischen Gleichgewichts im Sinne einer Dissoziation mit Hin- und Rückreaktion stellt keinen Nachteil dar.

Die antimikrobielle Zusammensetzung hat sowohl Biofilm reduzierende als auch antimikrobielle Eigenschaften. Bei der Freisetzung antimikrobieller Komponenten spielt möglicherweise die Verteilung entlang des Konzentrationsgradienten in Richtung Wunde eine Rolle, wobei die Kationen mittels der ihnen anhaftenden Hydrathülle der Wunde Feuchtigkeit zuführen. Ein feuchtes Wundmilieu fördert den Heilungsprozess.

Die in der Emulsion vorhandene antimikrobielle Zusammensetzung enthält mindestens die folgenden Komponenten:
a) ionisches Silber und / oder Silbernitrat, und
b) ionisches Zink und / oder Zinknitrat und / oder Zinksulfat, und
c) Ethylendiamintetraessigsäure (EDTA) und / oder Tetra-Natrium-EDTA.

Bevorzugt enthält die antimikrobielle Zusammensetzung 5 bis 50 Gew.-% der unter a) aufgeführten Komponenten, besonders bevorzugt 10 bis 45 Gew.-% und ganz besonders bevorzugt 20 bis 40 Gew.-%.

Weiterhin enthält die antimikrobielle Zusammensetzung bevorzugt 3 bis 45 Gew.-% der unter
b) aufgeführten Komponenten, besonders bevorzugt 5 bis 40 Gew.-% und ganz besonders bevorzugt 9 bis 35 Gew.-%.

Schließlich enthält die antimikrobielle Zusammensetzung bevorzugt 20 bis 90 Gew.-% der unter c) aufgeführten Komponenten, besonders bevorzugt 25 bis 60 Gew.-%.

Diese Konzentrationsangaben können sich sowohl auf die ionischen Formen als auch ausschließlich auf die Salze (z.B. Silbernitrat und Zinknitrat) beziehen. Das Zinknitrat kann im Ausgangszustand als Hexahydrat Zn(NO3)₂x6H₂O vorliegen. Tetra-Natrium-EDTA kann im Ausgangszustand als Tetrahydrat vorliegen.

Innerhalb der antimikrobiellen Zusammensetzung können die einzelnen Komponenten bevorzugt die folgenden Konzentrationsbereiche einnehmen: EDTA kann in einer Konzentration von 15 bis 90 Gew.-% oder aber 25 bis 80 Gew.-% vorhanden sein. Bevorzugt beträgt die Konzentration von EDTA mindestens 15 oder mindestens 25 Gew.-%. Silber kann in einer Konzentration von 5 bis 50 Gew.-% oder aber 10 bis 40 Gew.-% vorhanden sein. Bevorzugt beträgt die Konzentration von Silber mindestens 5 oder mindestens 10 Gew.-%. Zink kann in einer Konzentration von 3 bis 45 Gew.-% oder aber 6 bis 40 Gew.-% vorhanden sein. Bevorzugt beträgt die Konzentration von Zink mindestens 3 oder mindestens 6 Gew.-%.

Gemäß einer bevorzugten Ausführungsform enthält die antimikrobielle Zusammensetzung 5 bis 45 Gew.-% einer der folgenden Komponenten: ionisches Silber, Silbernitrat, ionisches Zink, Zinknitrat oder Zinksulfat. Gleichzeitig oder auch unabhängig davon kann die antimikrobielle Zusammensetzung 15 bis 90 Gew.-% EDTA enthalten.

Gemäß einer anderen bevorzugten Ausführungsform enthält die antimikrobielle Zusammensetzung 35 bis 45 Gew.-% Silbernitrat, 20 bis 40 Gew.-% Zinknitrat und 15 bis 30 Gew.-% Tetra-Natrium-EDTA.

Empfohlen wird das Verhältnis der Komponenten in der antimikrobiellen Zusammensetzung derart einzustellen, dass sowohl Silber als auch Zink (oder deren Salze wie z.B. Nitrate) jeweils im Überschuss gegenüber EDTA vorliegen. Der Begriff "Überschuss" bezieht sich auf die Gewichtskonzentration, wobei hierdurch auch automatisch ein molarer Überschuss hinsichtlich der Stoffmengenkonzentration erreicht wird.

Es wird empfohlen die Komponenten der antimikrobiellen Zusammensetzung zunächst in einer polaren, ungiftigen Flüssigkeit zu verteilen und diese Flüssigkeit anschließend mit den übrigen Bestandteilen der Emulsion zu vermengen. Die Verwendung von Wasser wird hierbei empfohlen, besonders empfohlen wird deionisiertes oder destilliertes Wasser, da es keine Ionen (wie z.B. Na⁺) enthält, die mit den Komponenten der antimikrobiellen Zusammensetzung reagieren könnten. Gemäß einer bevorzugten Ausführungsform enthält die polare Flüssigkeit mit der antimikrobiellen Zusammensetzung freie Kationen von Silber und Zink. Dies kann dadurch erreicht werden, dass sowohl Silber als auch Zink jeweils im Überschuss in Relation zu EDTA vorliegen. Beispielsweise kann die Summe der Stoffmengen von Silber und Zink mindestens die dreifache Stoffmenge an EDTA betragen. Die freien Kationen stehen auf diese Weise für biochemische Reaktionen bereit. So hat sich gezeigt, dass Silber vorrangig eine Wirkung gegenüber pathogenen Einzellern zeigt. Zink ist in der Lage Immunzellen zu aktivieren. Durch seine Funktion als Cofaktor in diversen Transkriptionsfaktoren und enzymatischen Reaktionen fördert Zink den Wundverschluss und schützt Zellen darüber hinaus vor Apoptose, die durch oxidativen Stress oder Bakterientoxine eingeleitet wird. Durch die Bindung von Kalzium kann EDTA vorhandene Biofilme zersetzen und ebenso deren Entstehung unterdrücken.

Die Freisetzung der Kationen (Ag⁺ und Zn²⁺) aus der erfindungsgemäßen Wundkontaktschicht kann innerhalb von 24 h bei 1 bis 200 µg pro 100 cm² Substrat liegen (in 100 ml Wasser, über 24 h, bei 37°C). Bevorzugt liegt die Freisetzung bei 10 bis 180 µg, noch besser bei 20 bis 160 µg oder bei 30 bis 100 µg . Der Wert kann eingestellt werden durch die Zusammensetzung der Emulsion, die Verhältnisanteile innerhalb der antimikrobiellen Zusammensetzung, den Wasseranteil und die Beschichtungsmenge der Emulsion auf dem Substrat. Im Allgemeinen gilt je polarer und hydrophiler die Emulsion und je höher ihr Wasseranteil, desto schneller werden die Kationen (und EDTA) freigesetzt. Details zu möglichen Beschichtungsmengen werden an anderer Stelle erläutert. Eine sehr schnelle Freisetzung kann nachteilig sein, da eine übermäßige Anreichung von Silberionen im menschlichen Gewebe einen cytotoxischen Effekt hervorrufen kann. Da bakterielle Zellen empfindlicher auf Silber reagieren als tierische, ist eine gemäßigte Silberfreisetzung im Bereich von 50 bis 90 µg (Bedingungen wie oben angegeben) häufig die beste Lösung.

Die Freisetzung der in der Emulsion enthaltenen antimikrobiellen Zusammensetzung kann unter Verwendung einer simulierten Wundflüssigkeit gemessen werden. Hierbei kann die gemessene Freisetzung innerhalb von 24 h bei 20 bis 60 mg pro Kilogramm Emulsion betragen. Die 20 bis 60 mg sind hierbei die Summe der Masse aus Kationen (Ag⁺, Zn²⁺) und EDTA. Eine geeignete Messmethode und die Zusammensetzung der Wundflüssigkeit sind in den Beispielen dargelegt.

Der Gehalt der antimikrobiellen Zusammensetzung in der Emulsion kann 0,5 bis 5 Gew.-% der Emulsion betragen. Bevorzugt enthält die Emulsion 0,5 bis 4 Gew.-%, besonders bevorzugt 0,8 bis 3,6 Gew.-% der antimikrobiellen Zusammensetzung. Weiterhin kann die Emulsion 0,2 bis 4,5 Gew.-% oder 0,4 bis 4 Gew.-% oder 0,6 bis 3,8 Gew.-% der antimikrobiellen Zusammensetzung enthalten.

Am besten enthält die Emulsion 1,3 bis 2,5 Gew.-% der antimikrobiellen Zusammensetzung, da hierbei in den meisten Fällen ein sehr guter Kompromiss aus Produkthaltbarkeit, antimikrobieller Wirkung und Wundheilung erzielt werden kann. Dieser Vorteil besteht auch im Hinblick auf die folgenden Konzentrationsangaben:
In Bezug auf die Salze der Komponenten der antimikrobiellen Zusammensetzung (Tetra-Natrium-EDTA, Zinknitrat oder Zinksulfat sowie Silbernitrat) hat sich eine Konzentration von 1,6 bis 3,7 Gew.-% oder noch besser 1,7 bis 3,6 Gew.-% dieser Komponenten in der Emulsion als besonders vorteilhaft erwiesen.

In Bezug auf die reinen Formen der Koponenten der antimikrobiellen Zusammensetzung (ionische Formen anstelle von Salzen) hat sich eine Konzentration von 0,8 bis 2,1 Gew.-% oder noch besser 0,9 bis 2 Gew.-% dieser Komponenten in der Emulsion als besonders vorteilhaft erwiesen.

Zusätzlich können die Komponenten der antimikrobiellen Zusammensetzung in einem bestimmten Mengenverhältnis zueinander stehen. So kann das Gewichtsverhältnis der Gewichtskonzentration von EDTA zur Summe der Gewichtskonzentrationen Ag⁺ und Zn⁺ in der Emulsion von 9 zu 1 bis hin zu 9 zu 36 reichen. Alternativ kann das Stoffmengenverhältnis der Stoffmenge von EDTA zur Summe der Stoffmengen aus Ag⁺ und Zn⁺ von 4 zu 1 bis hin zu 4 zu 32 reichen. Die Stoffmengenbestimmung kann in der Standardeinheit mol vorgenommen werden. Das Verhältnis der Komponenten zueinander ist ein Faktor, der darüber mitentscheidet, wie groß der Anteil der freien Kationen (Ag⁺, Zn⁺) zu den komplexierten und damit an EDTA gebundenen Kationen ist. Dabei hat sich überraschend gezeigt, dass sowohl freie Kationen als auch freie Bindungsstellen am EDTA-Molekül vorteilhaft sind. Vermutet wird, dass eben diese primär für die gewünschten Reaktionen gegen Erreger und Biofilme zur Verfügung stehen. Bevorzugte daraus abgeleitete Bereiche ist ein Gewichtsverhältnis der Gewichtskonzentration von 9 zu 30 bis hin zu 9 zu 36 (9 steht für Teile von EDTA) und ein Stoffmengenverhältnis der Stoffmenge von 4 zu 28 bis hin zu 4 zu 32 (4 steht für Teile von EDTA).

Der Gehalt an polarer Flüssigkeit bzw. an polaren Flüssigkeiten in der Emulsion kann an den Gehalt der antimikrobiellen Zusammensetzung in der Emulsion angepasst werden. Empfohlen wird mit zunehmender Konzentration an antimikrobieller Zusammensetzung den Gehalt an polarer Flüssigkeit zu steigern. So liegt bei einer Konzentration an antimikrobieller Zusammensetzung von 0,9 bis 1,5 Gew.-% der Flüssigkeitsgehalt bevorzugt bei 7 bis 11 Gew.-%, bei einer Konzentration an antimikrobieller Zusammensetzung von 1,6 bis 2,2 Gew.-% bevorzugt bei 12 bis 16 Gew.-% und bei einer Konzentration an antimikrobieller Zusammensetzung von 2,3 bis 2,7 Gew.-% bei bevorzugt 17 bis 20 Gew.-%. Diese Angleichung der Konzentrationen wirkt sich vorteilhaft auf die Stabilität der Emulsion aus.

Das netzförmige Substrat kann teilweise oder vollständig mit der Emulsion beschichtet sein. Die Beschichtung kann auf einer Seite oder auf beiden Seiten vorhanden sein. Falls nur eine Seite beschichtet ist, sollte vorzugsweise die zum Wundkontakt vorgesehene erste Seite beschichtet sein. Insbesondere können mindestens 80 % der Fläche der ersten Seite des Substrats mit der Emulsion beschichtet sein. Bevorzugt sind 90 % der Fläche der ersten Seite des Substrats mit der Emulsion beschichtet. Besonders empfohlen wird mindestens 99 % dieser Fläche mit der Emulsion zu beschichten. Hierbei werden Öffnungen im Substrat bei der Flächenbestimmung nicht berücksichtigt. Typischerweise wird die Emulsion ausreichend zähflüssig sein, um diese Öffnungen zu überdecken. Bei einem rechteckigen, netzförmigen Substrat in der Größe 6 cm x 10 cm wären somit 100 % des Substrats mit Emulsion beschichtet, wenn die Emulsion eine Fläche von 60 cm² bedeckt.

Nach dem Auflegen auf eine Wunde verteilt sich die Emulsion üblicherweise im Wundbereich. Ebenso kann die Emulsion bei exsudierenden Wunden verflüssigt werden, so dass in aller Regel zumindest ein Teil der zuvor beschichteten Öffnungen freigelegt wird, was dem Stoffaustausch zugutekommt.

Ein beispielhafter Beschichtungsprozess umfasst die folgenden Schritte:
1) Bereitstellung von Träger, Walze und Emulsion, wobei die Emulsion als Emulsionsbad vorliegt,
2) Eintauchen der Walze in das Emulsionsbad,
3) Drehen der Walze im eingetauchten Zustand, um diese vollständig mit Emulsion zu beladen,
4) Übertragen der Emulsion auf den Träger durch Rollen mit der Walze über diejenige Seite des Trägers, welche zum Auflegen auf eine Wunde vorgesehen bzw. konfiguriert ist oder durch Ziehen der besagten Seite des Trägers über die Walze.

Bei Bedarf kann der Prozessschritt 1) erweitert werden, indem die Emulsion im Emulsionsbad zusätzlich erwärmt wird (z.B. auf eine Temperatur zwischen 35 °C und 50 °C).

Die Beschichtung kann beispielsweise mittels einer sich drehenden Walze erfolgen, deren Oberfläche zunächst die Emulsion aufnimmt und diese anschließend auf das Substrat überträgt. Die Walze kann beladen werden, indem sie in die Emulsion eingetaucht und im eingetauchten Zustand gedreht wird. Alternativ kann das Substrat (welches üblicherweise als Massenware beim Herstellungsprozess vorliegt) über die sich drehende Walze geführt und dabei in die Emulsion eingetaucht werden. Dies führt normalerweise zu einer beidseitigen Beschichtung. In Abhängigkeit von der Formulierung der Emulsion und der Umgebungstemperatur kann es vorteilhaft sein, die Emulsion hierbei zu erwärmen. Ein Sprühen der Emulsion auf das Substrat ist möglich, solange die Emulsion nicht zu viskos ist. Bei zu hoher Viskosität kann die Emulsion vor dem Sprühvorgang erhitzt werden (z.B. auf 40 bis 50 °C), um die Viskosität herabzusetzen.

Die resultierende Beschichtungsmenge kann durch Wiegen des behandelten Substrats ermittelt werden. Beschichtungen mit größerem Flächengewicht können durch wiederholte Einzelbeschichtungen erzeugt werden.

Gemäß einer bevorzugten Ausführungsform der Erfindung umfasst die Wundkontaktschicht ein netzförmiges Substrat, dessen erste und zweite Seite mit der Emulsion beschichtet ist. Dies bietet den Vorteil, dass der Anwender nicht entscheiden muss, welche Seite zum Auflegen auf die Wunde konfiguriert ist, was die Wahrscheinlichkeit von Fehlern reduziert und die Arbeit im klinischen Alltag erleichtert.

Das netzförmige Substrat der Wundkontaktschicht kann mindestens eines der folgenden Materialien enthalten oder daraus bestehen: Polyamid, insbesondere Nylon, Polyester, Baumwolle, Viskose oder eine Kombination aus zwei oder mehr der aufgeführten Materialien. Die besagten Materialien können in Form von Fasern vorliegen. Beispielsweise kann es sich um Polyamidfasern, insbesondere Nylonfasern, Polyesterfasern, Baumwollfasern oder Viskosefasern handeln. Das Substrat kann dabei in Form eines Polyamidgewirkes vorliegen, insbesondere kann es als Nylongewirke vorliegen. Darüber hinaus können auch Mischfasern verwendet werden, welche eine Kombination aus zwei oder mehr der aufgeführten Materialien in sich vereinen. Ein Substrat in Form von Fasern lässt sich mit besonders großen Mengen an Emulsion beladen, da die Emulsion die Faserzwischenräume ausfüllen kann.

In vorteilhafter Weise und zur Erzielung einer besonders ausgeprägten antimikrobiellen Wirksamkeit kann die antimikrobiellen Zusammensetzung 35 bis 45 Gew.-% Silbernitrat, 20 bis 40 Gew.-% Zinknitrat und 15 bis 30 Gew.-% Tetra-Natrium-EDTA enthalten. Alternativ können die Komponenten im folgenden Gew.-Verhältnis zueinander in der antimikrobiellen Zusammensetzung vorliegen: 1,6 bis 2 Silbernitrat zu 2,9 bis 3,3 Zinknitrat zu 0,8 bis 1,2 Tetra-Natrium-EDTA.

Die Emulsion kann 0 bis 40 Gew.-% Wasser, bevorzugt 5 bis 40 Gew.-% Wasser oder 5 bis 30 Gew.-% Wasser, besonders bevorzugt 10 bis 30 Gew.-% Wasser enthalten und ganz besonders bevorzugt 20 bis 30 Gew.-% Wasser enthalten. Ein höherer Wasseranteil führt zu einer schnelleren Abgabe der antimikrobiellen Bestandteile. Ein niedrigerer Wasseranteil führt zu einer zeitverzögerten und längerfristigen Abgabe. Emulsionen mit einem niedrigerem Wasseranteil eignen sich besonders gut zur Prävention einer Infektion. Emulsionen mit höherem Wasseranteil werden empfohlen zur Behandlung bereits infizierter Wunden oder solcher Wunden, die von einem höheren Feuchtigkeitseintrag profitieren, wie z.B. Wunden mit Fibrinbelägen oder nekrotische Wunden.

Sowohl die erste und optional auch die zweite Seite des netzförmigen Substrats kann mit einer Emulsionsmenge von 100 bis 320 g/m², bevorzugt 110 bis 250 g/m², am besten 120 bis 200 g/m² beschichtet sein (Beschichtungsmenge). Es hat sich gezeigt, dass diese Menge zu hervorragenden Ergebnissen bei der Förderung der Wundheilung und der Bekämpfung von Krankheitserregern in der Wunde führt. Gleichzeitig ist es möglich ein stabiles Produkt mit ausreichend langer Lagerfähigkeit zu erhalten, und zwar ohne, dass sich die Emulsion von der Wundkontaktschicht löst. Bevorzugt handelt es sich bei der Beschichtung um eine gleichmäßige oder im Wesentlichen gleichmäßige Verteilung der Emulsion, bei der jeder Bereich der präparierten Seite der Wundkontaktschicht mit derselben Menge an Emulsion bestückt ist.

Alternativ kann bei einem mit Emulsion beschichteten netzförmigen Substrat das Verhältnis des Substratgewichts zum Emulsionsgewicht innerhalb folgender Spannen liegen: 1 zu 1,4 bis 1 zu 2,4, bevorzugt 1 zu 1,6 bis 1 zu 2,2 und besonders bevorzugt 1 zu 1,9 bis 1 zu 2,1.

Das netzförmige Substrat kann beispielsweise ein Flächgewicht von 50 bis 120 g/m², bevorzugt 70 bis 100 g/m² und besonders bevorzugt 80 bis 90 g/m² aufweisen. Derartige Substrate sind für gewöhnlich so dünn und / oder derart mit Öffnungen versehen, dass der Übergang von Flüssigkeit durch die Wundkontaktschicht in einen möglichen Sekundärverband optimiert ist. Darüber hinaus wird die Emulsion in einem solchen Substrat (im gegensatz zu dickeren oder dichteren Geweben) nicht nennenswert zurückgehalten, so dass die antimikrobiellen Komponenten und pflegenden Inhaltsstoffe effizient an Wunde und umgebende Haut abgegeben werden.

Weiterhin kann die lineare Dichte des netzförmigen Substrats 60 bis 100 dtex, bevorzugt 70 bis 90 dtex gemessen mittels DIN EN ISO 2060 betragen. Die Zähigkeit des Substrats kann 30 bis 50 cN/tex, bevorzugt 35 bis 40 cN/tex gemessen mittels DIN EN ISO 2062 betragen.

Weiterhin kann die Emulsion mindestens einen Bestandteil ausgewählt aus der folgenden Gruppe umfassen: Mono-, Di- und Triglyceride, Fettsäureester, Lanolin, ein Oligomer des Glycerins, Vaseline, Paraffin, synthetisches Wachs oder Mischungen daraus. Bei den Fettsäureestern kann es sich um Diglycerinester von Mono- und Dicarbonfettsäuren handeln. Mithilfe dieser Bestandteile kann eine Emulsion bereitgestellt werden, die sowohl atraumatische Eigenschaften aufweist - sich also leicht von der Wunde lösen lässt, ohne das Schmerzen verursacht werden - als auch überschüssige Feuchtigkeit bei exsudierenden Wunden aufnehmen oder Feuchtigkeit an trockene Wunden im Sinne einer feuchten Wundbehandlung abgeben kann. Insbesondere Wachsbestandteile (einschließlich Lanolin), Glycerinderivate und Fettsäureester können der Emulsion die Eigenschaft verleihen Feuchtigkeit aufzunehmen und abzugeben.

Bevorzugt umfasst die Emulsion ein Mono-, Di- oder Triglycerid, welches ein Mono-, Di- oder Triglycerid mindestens einer der folgenden Säuren ist: Caprylsäure, Caprinsäure, Isostearinsäure, Stearinsäure oder 12-Hydroxystearinsäure. Derartige Glyceride haben besonders gute emulgatorische Eigenschaften und lassen sich mit den übrigen Bestandteilen zu einer stabilen Emulsion vermischen. Darüber hinaus zeigen diese Glyceride hervorragende Lagerfähigkeit ohne die Neigung zu Oxidation oder anderen unerwünschten Reaktionen.

Ferner kann die Emulsion 3 bis 30 Gew.-% eines hydrophilen Bestandteils zusätzlich zu einem möglichen Wasseranteil enthalten. Wasser soll somit bei dem angegebenen Anteilsbereich unberücksichtigt bleiben. Dieser hydrophile Bestandteil ist eine hydrophile Verbindung und bevorzug eine kovalente (molekulare) hydrophile Verbindung. Sie kann in ihrer reinen Form als Flüssigkeit oder Feststoff (z.B. Pulver) vorliegen. Bevorzugt enthält die Emulsion 5 bis 15 Gew.-% der zusätzlichen hydrophilen Verbindung. Alternativ können bei dem angegebenen Anteilsbereich sämtliche polaren Flüssigkeiten (also zusätzlich zu Wasser auch Alkohole wie Glycerin) unberücksichtigt bleiben. Zu den zusätzlichen hydrophilen Verbindungen zählen wasserbindende Bestandteile und / oder Emulgatoren. Ein Beispiel für eine geeignete hydrophile Verbindung ist PEG. Durch Beimengung dieser zusätzlichen hydrophilen Verbindung können Stabilität, Konsistenz und Fließverhalten der Emulsion verbessert werden. Im Optimalfall sind diese Parameter so eingestellt, dass die Emulsion sich als stabile Beschichtung der Wundkontaktschicht auftragen lässt, aber bei Kontakt mit einer Wunde durch Einwirkung von Feuchtigkeit und Körperwärme ein Kriechverhalten zeigt, bei dem sie in die Wunde übertritt und sich in dieser verteilt.

Die zusätzliche hydrophile Verbindung kann Polymere, Alkohole, Glycerin oder Mischungen daraus umfassen. Mögliche Mischungen sind ein Alkohol mit Glycerin oder ein Polymer wie PEG (z.B. PEG 2000) mit einem Alkohol wie z.B. Ethanol.

Gemäß einer Variante ist oder enthält der ausgewählte hydrophile Bestandteil ein Polymer, bei dem es sich um ein Polysaccharid oder um ein PEG (z.B. PEG 2000) oder um eine Mischung aus diesen beiden Polymeren handelt.

Wenn es sich bei dem ausgewählten hydrophilen Bestandteil um ein Polymer handelt, das ein Polysaccharid ist, so ist oder umfasst dieses Polysaccharid Cellulose, Methylcellulose, Carboxymethylcellulose, Stärke, modifizierte Stärke, Alginate, Chitosan oder Mischungen daraus. Beispiele für modifizierte Stärke sind oxidierte Stärke, Monostärkephosphat, Distärkephosphat, phosphatiertes Distärkephosphat, acetyliertes Distärkephosphat, acetylierte Stärke, Hydroxypropylstärke und Dextrin. Unter diesen Bestandteilen sind Cellulose sowie die genannten Derivate der Cellulose und Chitin bevorzugt, da sie von den meisten pathogenen Mikroorganismen nicht als Kohlenstoffquelle (Nährsubstrat) genutzt werden können. Umgekehrt können niedermolekulare Polysaccharide (z.B. Dextrin) in ausreichender Konzentration ein starkes osmotisches Potential entfalten und auf diese Weise die Vermehrung von Mikroorganismen hemmen.

Sollte es sich bei dem ausgewählten hydrophilen Bestandteil um einen Alkohol handeln, so kann vorgesehen sein, dass dieser Alkohol Ethanol, einen Zuckeralkohol wie z.B. Glycerin, Propylenglykol, Ethylenglykol oder Mischungen daraus umfasst oder ist.

Weiterhin kann es sich bei dem ausgewählten hydrophilen Bestandteil um PEG handeln, wobei das PEG beispielsweise ein Molekulargewicht von 0,2 bis 8 kDa, bevorzugt 1 bis 3 kDa, besonders bevorzugt 2 kDa (PEG 2000) hat. Weiterhin kann das PEG eine maximale Molmasse von 600 g/mol aufweisen, wobei PEG mit einer solchen maximalen Molmasse als polare Flüssigkeit vorliegt. Das PEG wirkt auf der Haut oder der Wunde nicht okklusiv, sondern steigert aufgrund der osmotischen Aktivität die Sekretaufnahme in die Emulsion und damit auch in die Wundkontaktschicht. Je niedriger die molare Masse des PEG desto stärker ist dieser Effekt ausgeprägt. Gleichzeitig wirkt PEG in der Emulsion als feuchtigkeitsbindender Faktor, der sogar in der Lage ist, einer vorzeitige Austrocknung der Emulsion entgegenzuwirken.

Das netzförmige Substrat kann mit elementarem, nicht ionischem Silber beschichtet oder imprägniert sein. In diesem Fall besteht diese Silberbeschichtung zusätzlich zu dem in der antimikrobiellen Zusammensetzung enthaltenen Silberanteil. Die zusätzliche Silberbeschichtung kann die antimikrobielle Wirkung weiter steigern, ermöglicht eine Depotwirkung und kann darüber hinaus die Fähigkeit von Krankheitserregern sich im Substrat anzusiedeln und zu vermehren herabsetzen oder sogar ganz aufheben. Letzteres ist besonders von Vorteil bei infizierten Wunden, die erregerhaltiges Exsudat abgeben, welches von der Wundkontaktschicht aufgenommen wird.

Der Tropfpunkt der Emulsion kann gemäß einer Bestimmung nach der Norm DIN ISO 2176 oberhalb von 35°C, bevorzugt zwischen 35°C und 45°C und besonders bevorzugt zwischen 35°C und 37°C liegen. Eine Möglichkeit den Tropfpunkt einzustellen, ist die Zugabe bestimmter unpolarer, aliphatischer Kohlenwasserstoffe, Öle oder Fettsäuren zu der Emulsion. Hierbei setzen langkettige, gesättigte Verbindungen den Tropfpunkt herauf und kurzkettige bzw. ungesättigte Verbindungen setzen den Tropfpunkt herab. Ein Tropfpunkt, der sich im ungefähren Bereich der Körpertemperatur befindet, bietet den Vorteil, dass die Emulsion sich unter Einwirkung der Körperwärme besser ausbreitet und dabei in die Wunde übergeht. Der Tropfpunkt sollte jedoch nicht niedriger gewählt werden als nötig, da sich dies ansonsten ungünstig auf die Stabilität und Lagerungsfähigkeit der behandelten Wundkontaktschicht auswirken kann.

Die Wundkontaktschicht kann eine Dehnbarkeit nach DIN EN ISO 1798:2008-04 von mindestens 25%, bevorzugt von mindestens 35% haben. Dehnbarkeit ist dahingehend zu verstehen, dass es bei einer entsprechenden Verlängerung des Materials unter Zugkraft nicht zu einem Belastungsbruch oder zu einem Reißen von Fasern kommt. Bevorzugt ist die Dehnbarkeit eine reversible Dehnbarkeit, so dass das Material nach Aufhebung der Zugkraft im Wesentlichen seine ursprüngliche Länge einnimmt. Eine reversible Dehnbarkeit liegt auch dann vor, wenn das Material nach Aufhebung der Zugkraft eine Länge einnimmt, die maximal 105% der Ausgangslänge entspricht. Die Länge hat dabei dieselbe räumliche Ausrichtung wie die Zugkraft, ist also in Richtung der Zugkraft zu messen. Bevorzugt gelten die angegebenen Dehnungswerte in Faserrichtung. Eine definierte Faserrichtung ist bei den meisten textilen Anordnungen gegeben.

Die Freisetzung der Kationen (Ag⁺ und Zn²⁺) aus der Emulsion, mit welcher das Substrat beschichtet ist, beträgt innerhalb von 24 h jeweils 1 bis 200 µg pro 100 cm² beschichtetem Substrat, wobei die Freisetzung an ein Wasservolumen von 100 ml Wasser, innerhalb eines Zeitraums von 24 h und bei einer Temperatur von 37°C erfolgt. Bevorzugt liegt die Freisetzung unter diesen Bedingungen bei 10 bis 180 µg, noch besser bei 20 bis 160 µg und am besten bei 30 bis 100 µg.

Die Erfindung betrifft darüber hinaus eine Wundauflage, die die erfindungsgemäße Wundkontaktschicht umfasst. Die Wundkontaktschicht liegt dabei als eine Außenschicht der Wundauflage vor, die während der Anwendung in Kontakt mit der Haut bzw. der Wunde steht. Vor der Verwendung kann die erste Seite der Wundkontaktschicht durch eine Schutzschicht wie beispielsweise einen Release-Liner überdeckt werden. Die Schutzschicht wird vor der Verwendung entfernt.

Bevorzugt umfasst diese Wundauflage eine Absorptionsschicht, welche auf der zweiten Seite der Wundkontaktschicht angebracht ist und / oder einen umlaufenden Kleberand zum Ankleben der Wundauflage an die Haut, welche die Wunde umgibt und / oder eine rückseitige (oberhalb der zweiten Seite der Wundkontaktschicht gelegene) Stützschicht (Backing). Die Absorptionsschicht kann Vliesgewebe, Superabsorber oder ein geschäumtes Material umfassen. Die Stützschicht kann als Folie ausgebildet sein, die optional durchlässig für Wasserdampf, aber nicht für flüssiges Wasser ist und die ein Polyurethan (PU) enthalten oder daraus bestehen kann. Der umlaufende Kleberand kann Teil der Stützschicht sein. Eine Absorptionsschicht bietet sich vor allem für blutende oder stark exsudierende Wunden an.

Eine Stützschicht hat den Vorteil, dass Wunde und Wundkontaktschicht vor Nässe von außen geschützt werden, beugt zudem einer Übertragung von Emulsion auf Kleidung oder Bettwäsche vor und trägt zum Erhalt der Feuchtigkeit (z.B. durch eine Stabilisierung des Wassergehalts) in der Emulsion bei.

Ein weiterer Aspekt der Erfindung betrifft die erfindungsgemäße Wundkontaktschicht oder eine Wundauflage, die diese Wundkontaktschicht umfasst, zur Anwendung in einem Verfahren zur Behandlung von Wunden, bevorzugt von infizierten Wunden, besonders bevorzugt von infizierten, biofilmhaltigen Wunden.

Ein weiterer Aspekt der Erfindung betrifft eine Emulsion enthaltend eine antimikrobielle Zusammensetzung, wie vorliegend beschrieben, zur Anwendung in einem Verfahren zur Behandlung von Wunden, bevorzugt von infizierten Wunden, besonders bevorzugt von infizierten, biofilmhaltigen Wunden, wobei die Emulsion als Beschichtung auf einem netzförmigen Substrat, welches als Wundkontaktschicht vorgesehen ist, vorliegt.

Gemäß einer bevorzugten Ausführungsform der Erfindung ist der Träger der Wundkontaktschicht beschichtet mit einer Emulsion, die eine Wasserkonzentration von 20 bis 30 Gew.-% enthält, in Kombination mit einer antimikrobiellen Zusammensetzung, die mehr als 15 Gew.-% EDTA enthält. Dabei ist eine Konzentration von 15 bis 92 Gew.-% EDTA besonders bevorzugt und eine Konzentration von 15 bis 30 Gew.-% am meisten bevorzugt.

Gemäß einer anderen bevorzugten Ausführungsform der Erfindung ist der Träger der Wundkontaktschicht beschichtet mit einer Emulsion, die eine Wasserkonzentration von 26 bis 29 Gew.-% enthält, in Kombination mit einer Konzentration der antimikrobiellen Zusammensetzung in der Emulsion von 1 bis 2 Gew.-%. Dabei ist eine Konzentration von 1,2 bis 1,6 Gew.-% der antimikrobiellen Zusammensetzung besonders bevorzugt.

Eine besonders bevorzugte Ausführungsform betrifft eine erfindungsgemäße Wundkontaktschicht wobei das netzförmige Substrat ein Tüllgewebe ist, das Polyester oder Polyamid enthält und wobei die Emulsion 1,3 bis 1,5 Gew.-% der antibakteriellen Zusammensetzung sowie 26 bis 29 Gew.-% Wasser und darüber hinaus Triglyceride und PEG enthält und wobei die antibakterielle Zusammensetzung 43 bis 49 Gew.-% Silber, 24 bis 30 Gew.-% Zink und 24 bis 28 Gew.-% EDTA enthält. Die Angabe "1,3 bis 1,5 Gew.-% der antibakteriellen Zusammensetzung" kann sich dabei auch ausschließlich auf die ionischen Formen der Komponenten der antibakteriellen Zusammensetzung beziehen.

Weiterhin können die Merkmale der beiden zuletzt genannten Ausführungsformen miteinander kombiniert werden. Diese besagten Merkmale der beiden zuletzt genannten Ausführungsformen erlauben eine ausgezeichnete Wirkung gegen den gefährlichen Keim S. *aureus* und zwar auch gegen die besonders problematischen MRSA-Varianten.

Daneben betrifft die Erfindung auch die Verwendung der Wundkontaktschicht zur Herstellung der oben beschriebenen Wundauflage oder die Verwendung der beschriebenen Emulsion enthaltend die antimikrobielle Zusammensetzung in einem Verfahren zur Herstellung der Wundkontaktschicht. Ein mögliches Herstellungsverfahren wird nachfolgend erläutert.

Teil der Erfindung ist ein Verfahren zur Herstellung einer Wundkontaktschicht wie hierin beschrieben, umfassend die folgenden Schritte:
(I) Bereitstellen einer antimikrobiellen Zusammensetzung durch Einbringen der folgenden Komponenten in eine polare Flüssigkeit:
   a) ionisches Silber oder Silbernitrat,
   b) ionisches Zink oder Zinknitrat oder Zinksulfat, und
   c) EDTA.
(II) Herstellen einer Emulsion, die die unter (I) bereitgestellte antimikrobielle Zusammensetzung beinhaltet.
(III) Beschichten zumindest eines Teils der ersten Seite eines netzförmigen Substrats mit der unter 2) erhaltenen Emulsion, wodurch die erste Seite des netzförmigen Substrats zum Auflegen auf eine Wunde konfiguriert wird. Es kann zusätzlich auch die zweite Seite beschichtet werden. Ebenfalls kann ein Beschichtungsverfahren eingesetzt werden, bei dem beide Seiten gleichzeitig beschichtet werden.

In einem optionalen Schritt (IV) kann mindestens die erste Seite der Wundkontaktschicht mit einer Schutzschicht oder Schutzfolie überlagert werden, die Wundkontaktschicht verpackt werden und die verpackte Wundkontaktschicht sterilisiert werden.

Ein weiterer Aspekt der Erfindung ist ein Kit enthaltend die erfindungsgemäße Wundkontaktschicht in Kombination mit einem Sekundärverband. Der Sekundärverband sollte in der Lage sein die Wundkontaktschicht an der Wunde zu fixieren. Dazu kann der Sekundärverband beispielsweise über Klebemittel verfügen oder über die Wundkontaktschicht und den betreffenden Körperbereich gewickelt werden.

Bei dem Kit kann es sich um eine Verpackung oder ein Set handeln.

Die erfindungsgemäße Wundkontaktschicht ist geeignet zur Abdeckung und / oder Therapie von Wunden, insbesondere infizierten Wunden und besonders zur Therapie solcher Wunden, die Biofilme aufweisen. Aus diesen Gründen kann die Wundauflage eingesetzt werden in einem Verfahren zur Wundtherapie und / oder zur Reduktion der Keimzahl in Wunden und / oder Wundbiofilmen.

### Figuren

Im Folgenden werden die Figuren näher erläutert. Wo Keimzahlreduktionen dargestellt sind, erfolgen diese anhand eines dekadisch-logarithmischen Maßstabs der Ordinate:

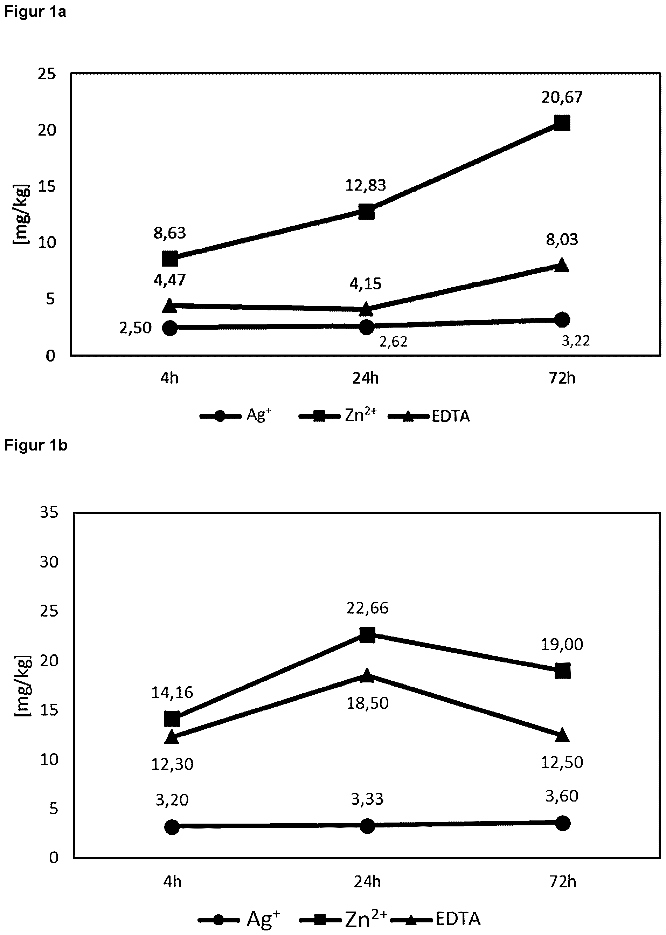

Fig. 1a zeigt die Freisetzungsrate der antimikrobiellen Zusammensetzung aus einer Emulsion mit etwa 1,4 Gew.-% dieser Zusammensetzung (bezogen auf die ionischen Formen) an simulierte Wundflüssigkeit über einen Zeitraum von insgesamt 72 h. Die animikrobielle Zusammensetzung in der Emulsion bestand aus 18 Gew.-% Tetra-Natrium-EDTA, 39 Gew.-% Silbernitrat und 43 Gew.-% Zinknitrat.
Fig. 1b zeigt die Freisetzungsrate der antimikrobiellen Zusammensetzung aus einer Emulsion mit 1,94 Gew.-% dieser Zusammensetzung (bezogen auf die ionischen Formen) an simulierte Wundflüssigkeit über einen Zeitraum von insgesamt 72 h. Die animikrobielle Zusammensetzung in der Emulsion bestand aus 18 Gew.-% Tetra-Natrium-EDTA, 39 Gew.-% Silbernitrat und 43 Gew.-% Zinknitrat.

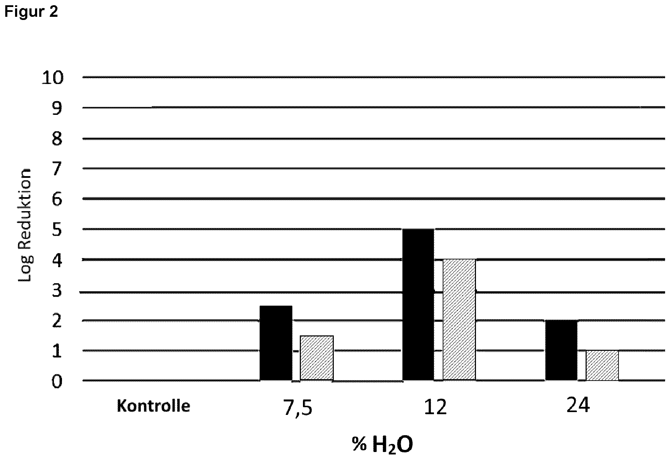

Fig. 2 zeigt die Reduktion von im CDC-Bioreaktor erzeugten Biofilmen von P. *aeruginosa* (ausgefüllte Balken) und S. aureus (schraffierte Balken) durch erfindungsgemäße Wundkontaktschichten in Abhängigkeit von der Wasserkonzentration der Emulsion bei einem Wirkstoffgehalt der antimikrobiellen Zusammensetzung in der Emulsion von etwa 0,94 Gew.-% (bezogen auf die ionischen Formen). An der Abszisse ist die Wasserkonzentration der Emulsion (7,5 , 12 und 24 Gew.-%) dargestellt. Dabei stehen ausgefüllte Balken für den Stamm P. *aeruginosa* (ATCC 15442) und schraffierte Balken für S. *aureus* (ATCC 6538).

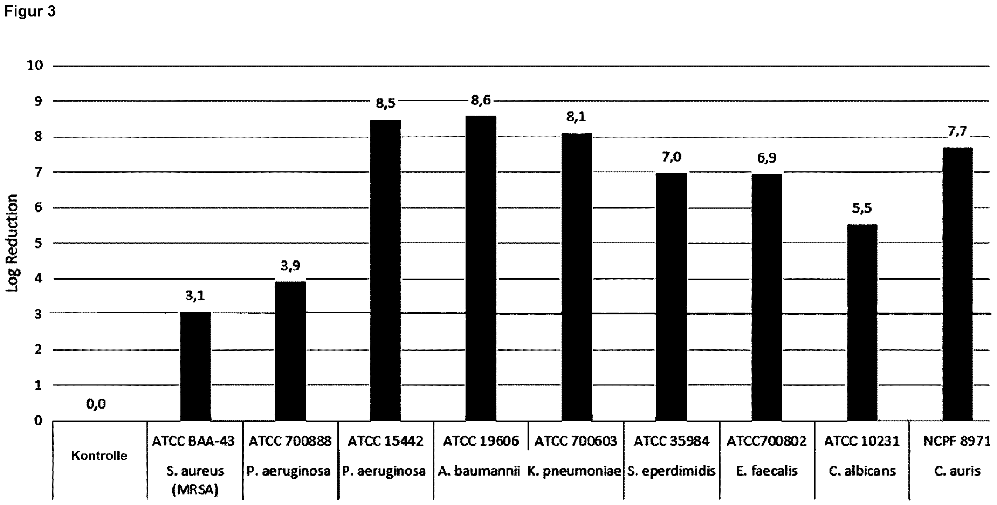

Fig. 3 zeigt die Reduktion von mittels Drip-Flow-Bioreaktor erzeugten Biofilmen verschiedener Organismen durch erfindungsgemäße Wundkontaktschichten mit etwa 1,94 Gew.-% der antimikrobiellen Zusammensetzung (bezogen auf die ionischen Formen) in der Emulsion. Die animikrobielle Zusammensetzung in der Emulsion bestand aus 18 Gew.-% Tetra-Natrium-EDTA, 39 Gew.-% Silbernitrat und 43 Gew.-% Zinknitrat.

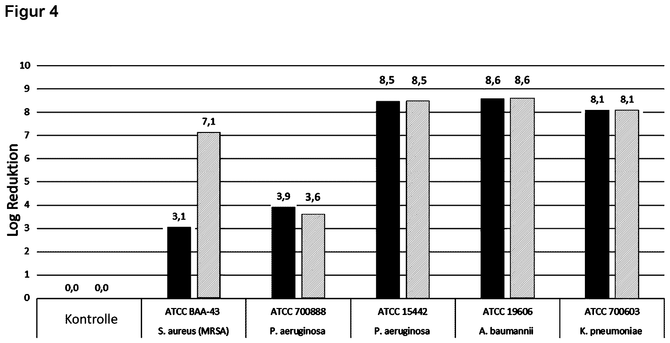

Fig. 4 zeigt den Wirksamkeitsvergleich zwischen der für Fig. 3 angegebenen Emulsion (ausgefüllte Balken) und einer Emulsion, die ebenfalls etwa 1,94 Gew.-% der antimikrobiellen Zusammensetzung enthielt (bezogen auf die ionischen Formen), allerdings in diesem Fall bestehend aus 86 Gew.-% Tetra-Natrium-EDTA, 6,5 Gew.-% Silbernitrat und 7,5 Gew.-% Zinknitrat (schraffierte Balken). Der Vergleich basiert auf einer Auswahl bestimmter Organismen aus Fig. 3.

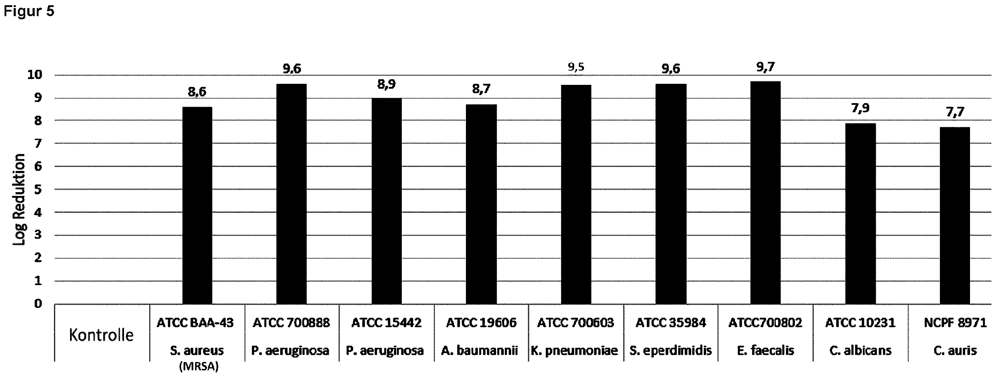

Fig. 5 zeigt die Reduktion der Zellzahl pathogener Organismen in einem Kontakttest nach Anlehnung an den Standard AATCC 100. Getestet wurden erfindungsgemäße Wundkontaktschichten mit etwa 1,94 Gew.-% antimikrobieller Zusammensetzung in der Emulsion (bezogen auf die ionischen Formen). Die antimikrobielle Zusammensetzung bestand aus 18 Gew.-% Tetra-Natrium-EDTA, 39 Gew.-% Silbernitrat und 43 Gew.-% Zinknitrat. Die Wundkontaktschichten wurden jeweils in dreifacher Ausführung getestet. Die dargestellten Werte sind Mittelwerte.

### Beispiele

### Beispiel 1: Emulsion mit antimikrobieller Zusammensetzung

Es wurden 42 g antimikrobieller Emulsion nach folgendem Ablauf hergestellt:

Zunächst wurde eine wässrige Lösung der antimikrobieller Zusammensetzung mit den folgenden Inhaltsstoffen angesetzt:

| | |
|---|---|
| Tetranatriumsalz von EDTA: | 800 mg |
| Silbernitrat: | 1.440 mg |
| Zinknitrat-Hexahydrat: | 2.480 mg |
| Wasser (desionisiert): | 38.210 mg |

29.670 mg dieser antimikrobiellen Lösung wurden anschließend mit den folgenden Bestandteilen zu 100 g Emulsion vermengt:

| | |
|---|---|
| Triglyceride: | 45.710 mg |
| Weitere unpolare Inhaltsstoffe: | 17.580 mg |
| PEG 2000: | 7.030 mg |

Als Triglyceride wurde eine Mischung aus gesättigten C₈ - C₁₈ Fettsäureestern pflanzlichen Ursprungs (Caprylic / Capric / Staearic Triglyceride) verwendet. Diese Mischung war bei Raumtemperatur zwar fest, jedoch mit anderen (unpolaren) Stoffen mischbar. Es hatte sich gezeigt, dass Körperwärme diese Mischung zum Schmelzen bringt, woraufhin sie hervorragende Kriecheigenschaften erhält und sich ausgezeichnet verteilt, ohne dass es hierzu zusätzlicher Maßnahmen bedürfte.

Darüber hinaus ist die Triglycerid-Mischung ungiftig und kann problemlos auf offene Wunden aufgebracht werden. Auf Wundränder übt die Mischung einen rückfettenden Effekt aus und verleiht ihnen einen hydrophoben Überzug, der einer Mazeration der Wundränder durch Wundexsudat entgegenwirkt.

Als weitere unpolare Inhaltsstoffe kamen Partialester des Diglycerols zum Einsatz. Dabei waren die folgenden Verbindungen mit dem Diglycerol verestert: Fettsäuren mittlerer Kettenlänge, Isostearinsäure, Stearinsäure, Adipinsäure und 12-Hydroxystearinsäure. Besagte Diglycerolester können mehr als 200 Gew.-% an Wasser aufnehmen. Es hat sich gezeigt, dass die Emulsion nach Beimischung dieser Diglycerolester eine deutlich gesteigerte Stabilität bei höheren Temperaturen hat. Gleichzeitig wurde die Durchlässigkeit für Wundexsudat gesteigert.

Als Ausgangsmaterial zur Bereitstellung des Substrats wurde ein Tüllgewebe aus Polyester gewählt. Die lineare Dichte nach DIN EN ISO 2060 betrug 76 dtex. Aus diesem Material wurde eine Fläche von 20 cm x 10 cm ausgeschnitten. Diese ausgeschnittene Fläche hatte ein Gewicht von 1,6 g und diente im weiteren Verlauf als Substrat für die Wundkontaktschicht. Das Substrat wurde mittels Dip Coatings mit 2,56 g der Emulsion versehen. Dies entsprach einem Beschichtungsmenge von 128 g / m².

Die Komponenten der antimikrobiellen Zusammensetzung bestanden aus den Salzen Tetranatrium-t-EDTA, AgNO₃ sowie Zn(NO₃)₂ und machten 3,26 Gew.-% der Emulsion aus. In Bezug auf die Reinkomponenten der antimikrobiellen Zusammensetzung bestehend aus EDTA, Ag⁺ und Zn²⁺ ergibt sich hiermit ein relevanter Wirkstoffanteil von 1,27 Gew.-% entsprechend 1,27 g von 100 g Emulsion. Davon waren 423 mg EDTA, 494 mg Zink- und 356 mg Silber-Ionen. Der Wasseranteil in der Emulsion betrug 27 Gew.-%. Der Öl-Anteil bzw. Anteil fettlöslicher Bestandteile in der Emulsion betrug 63,29 Gew.-%.

### Beispiel 2: Freisetzung der antimikrobiellen Zusammensetzung

Zum Nachweis der Freisetzung der antimikrobiellen Zusammensetzung aus einer mit Emulsion beschichteten erfindungsgemäßen Wundauflage wurde die Abgabe an simulierte Wundflüssigkeit mittels folgendem Szenario bestimmt:
Bereitgestellt als Substrat in ausreichender Stückzahl wurden identische Tüllgewebe a) und b) aus einem Gewirke von Polyethylenterephthalat mit einer Fläche von jeweils 3 cm x 3 cm und einer Dichte von 1,3 - 1,4 g/cm³. Die Gewebe wurden auf einer Seite mit erfindungsgemäßer Emulsion beschichtet, wobei die Emulsion auf Gewebe a) 1,4 Gew.-% an antimikrobieller Zusammensetzung enthielt und die Emulsion auf Gewebe b) 1,94 Gew.-% an antimikrobieller Zusammensetzung (bezogen auf die ionischen Formen). Die antimikrobielle Zusammensetzung bestand aus 26 Gew.-% EDTA, 46 Gew.-% Ag⁺ und 28 Gew.-% Zn²⁺. Die übrigen Bestandteilte der Emulsion waren analog zu der Formulierung aus Beispiel 1.

Anschließend wurden zweimal 10 ml simulierter Wundflüssigkeit (SWF) angerührt. Diese bestand zu 50 Vol.-% aus Rinderserum ("bovine serum") und zu 50 Vol.-% aus wässriger Pepton-Lösung. Die Pepton-Lösung enthielt die folgenden Inhaltsstoffe pro 1 I Nährmedium: 3,56 g Kaliumhydrogenphosphat, 5,77 g Dinatriumhydrogenphosphat, 4,3 g NaCl, 1g Caseinpepton (pankreatisch verdaut).

Tüllgewebe a) und b) wurden in jeweils 10 ml SWF in verschlossenen Flaschen (20 ml Volumen) bei 37 °C auf einem Schüttler inkubiert. Die Probenentnahme erfolgt nach 4 h, 24 h und 72 h. Gewebe und SWF wurden während der gesamten Inkubationsdauer in Bewegung gehalten, so dass die Emulsion kontinuierlich benetzt wurde.

Die entnommenen Proben wurden mittels gemäß EN 13368-1 auf eluiertes EDTA analysiert (jeweils 4 ml SWF pro Zeitpunkt). Dazu wurde das in die SWF abgegebene EDTA mit Fe(III) komplexiert und der resultierende Komplex mittels HPLC-UV detektiert (Absorptionsmaximum bei 260 nm).

Für die Analyse der in die SWF freigesetzten Kationen (Ag, Zn) wurden Probenfläschchen nach denselben drei Inkubationszeiten vom Schüttler entnommen, und die Wundauflagen aus den Probenfläschchen entfernt. Die 10 ml SWF wurden mit 20µl HNO₃ (65 Gew.-% in H₂O) versetzt, um die Silberionen vor unerwünschtem Ausfall zu schützen. Anschließend erfolgte die Bestimmung der Kationenkonzentration nach DIN EN ISO 11885 mittels coupled plasma optical emission spectrometry (ICP-OES).

Die Ergebnisse sind in Fig. 1a (1,4 % antimikrobielle Zusammensetzung in der Emulsion) und Fig. 1b (1,94 % antimikrobielle Zusammensetzung in der Emulsion) dargestellt.

### Beispiel 3: Nachweis der Wirksamkeit erfindungsgemäßer Wundkontaktschichten gegenüber Biofilmen mittels eines CDC Biofilm-Reactors^{®}

Der Test wurde unter Verwendung eines Testsystems, nämlich dem CDC Biofilm-Reactor^{®} (erhältlich bei Biosurface Technologies Corporation, USA), durchgeführt. Der CDC Biofilm-Reactor^{®} wird im Folgenden als "Reaktor" bezeichnet. Ziel des Tests war die Erzeugung eines Biofilms und die Messung der antimikrobiellen Wirksamkeit erfindungsgemäßer Wundkontaktschichten gegenüber einem solchen Biofilm. Anhand eines derartigen Tests kann die Wirksamkeit von Substanzen, welche einen Biofilm in Wunden bekämpfen sollen, simuliert werden.

Der verwendete Reaktor verfügte in seinem Inneren über acht Stäbe, an denen jeweils drei Proben befestigt wurden. Der Reaktor-Behälter fasste somit vierundzwanzig Proben. Diese Proben wurden in Form sogenannter Coupons montiert. Bei den Coupons handelte es sich um runde Träger mit Oberflächen, die die Anhaftung von Mikroorganismen und deren Zusammenschluss zu einem Biofilm ermöglichten.

Während der Versuchsdurchführung wurden Nährstoffe für die Zellproliferation kontinuierlich und in konstanter Menge in den Reaktor eingeleitet und wieder abgeführt, so dass ein gleichbleibendes Volumen vorherrschte.

Als Protokoll diente eine leicht modifizierte Fassung der Norm ASTM E2871 - 13:
Es wurden Übernachtkulturen der beiden Bakterienspecies S. *aureus* (Stamm ATCC 6538) und P. *aeruginosa* (Stamm ATCC 15442) angesetzt. Dazu wurden jeweils 10 ml TSB-Medium (tryptic soy broth) mit einer Einzelkolonie angeimpft und bei 37 °C auf einem Orbitalschüttler inkubiert.

Die Übernachtkultur von P. *aeruginosa* wurde auf 1x10⁸ CFU / ml (colony forming units) eingestellt. 1 ml dieser Kultur wurde anschließend genutzt, um 300 ml TSB zu inokulieren. Dieses Medium wurde in den Reaktor überführt und in diesem die weitere Kultvierung als Batch-Ansatz durchgeführt, und zwar bei 37 °C und 80 rpm auf einer Magnet-Rührplatte.

Nach einem 24 stündigem Biofilm-Wachstum wurden die Stäbe zwei Mal mit PBS-Medium (phosphate-buffered saline) gewaschen und die nun Biofilm-haltigen Coupons in 12-Well-Platten überführt. Dabei wurde jeder Coupon mit Proben der erfindungsgemäßen Wundkontaktschicht (2,5 cm x 5 cm) umwickelt. Die anschließende Inkubation dauerte 24 h und wurde bei Raumtemperatur (P. *aeruginosa)* bzw. 37 °C *(S. aureus)* durchgeführt. Anschließend wurden die Coupons aus den Well-Vertiefungen entnommen, in 10 ml Dey Engley neutralising broth überführt und 30 min lang einer Ultraschallbehandlung unterzogen.

Die Proben wurden mittels eines Vortex-Gerätes kurz geschüttelt und auf 96 Well-Platten verteilt. Es folgte eine serielle Verdünnung jeder Probe (1:10 in PBS), welche anschließend auf TSA ausplattiert wurden. Dabei wurden 20 µl pro Verdünnung eingesetzt (zweifacher Ansatz für jede Verdünnung jeder Probe). Die Platten wurden bei 37 °C über Nacht inkubiert. Am folgenden Tag erfolgte die Auszählung der Kolonien.

Die Ergebnisse sind in Fig. 2 dargestellt für Wundkontaktschichten mit einer Konzentration an antimikrobieller Zusammensetzung von 0,94 Gew.-% (bezogen auf die ionischen Formen).

### Beispiel 4: Nachweis der Wirksamkeit erfindungsgemäßer Wundkontaktschichten gegenüber Biofilmen mittels Drip-Flow-Bioreaktor

Weitere Tests wurden an solchen Biofilmen durchgeführt, die im Drip-Flow-Reaktor erzeugt wurden. Die Analyse mittels Drip-Flow-Reaktor findet unter Luftzugang statt und hat größere Ähnlichkeit mit den Bedingungen, wie sie in einer Wunde vorherrschen. Diese Tests fanden in Anlehnung an den Standard ASTM E2647 - 13 statt. Allerdings mit minimalen Abwandlungen, um Daten ausgehend von verschiedenen Mikroorganismenspezies zu generieren. Die eingesetzten Organismen lassen sich der folgenden Tabelle entnehmen:

**Tabelle I**

| **grampositive Organismen** | | **Hinterlegungs-Nr. bzw. Stamm** |
|---|---|---|
| | Staphylococcus aureus | ATCC 29213, MRSA ATCC BAA-43 |
| | Staphylococcus epidermidis | ATCC 35984 |
| | Enterococcus faecalis | ATCC 29212 |

| g**ramnegative Organismen** | | |
|---|---|---|
| | Pseudomonas aeruginosa | ATCC 15442 |
| | | ATCC 700888 |
| | Acinetobacter baumannii | ATCC 19606 |
| | Klebsiella Pneumoniae | ATCC 700603 |
| | | |

| **pilzliche Organismen** | | |
|---|---|---|
| | Candida albicans | ATCC 10231 |
| | Candida auris | NCPF 8971 |

Zunächst wurden 10 ml TSB mit einer einzelnen Kolonie eines jeden Stammes angeimpft und bei 37 °C und 125 rpm auf einem Schüttelinkubator als Übernachtkultur angesetzt. Absorbierende Pads wurden mittels Klebstoff an den Objektträgern befestigt und die Kammern des Drip Flow Reaktors mit den Objektträgern bestückt. Anschließend wurde der Reaktor im Autoklaven sterilisiert.

Die absorbierenden Pads wurden mit 1 ml TSB befeuchtet und mit Polycarbonatfiltern bestückt (2 cm x 2 cm ; 0,2 µm). Die Übernachtkulturen wurden auf 1 x 10⁸ CFU/ml eingestellt. Anschließend wurden die Filter mit 10 µl dieser Lösung benetzt und trocknen lassen.

Die Abdeckungen des Reaktors wurden befestigt und der Reaktor mit einem 10 l Glasballon (270 mg/l TSB) befestigt. Der Schlauch für die Nährstofflösung wurde an eine Pumpe angeschlossen und die Nährstoffe mit einer Rate von 5 ml/min/Kammer zugeführt.

Nach 24 h wurden die erfindungsgemäßen Wundkontaktschichten auf eine Größe von 2,5 cm x 2,5 cm zugeschnitten und vollständig mit simulierter Wundflüssigkeit gesättigt (50 : 50 - "maximum recovery diluent": fötales Kälberserum). Die Wundkontaktschichten wurden anschließend auf die Oberseite der inokulierten Filter aufgelegt, die Abdeckungen der Kammern gesichert und der Reaktor abermals für 24 h in Betrieb genommen.

Nach Ablauf der Zeit wurden die Filter entnommen, in 10 ml "Dey-Engley neutralizing broth" gegeben und für 30 min sonifiziert. Die Proben wurden auf 96-Well Platten verteilt und eine 1:10 Verdünnungsreihe aus jeder Probe mit PBS generiert. Die Verdünnungen wurden auf TSA ausplattiert (20 µl). Nach Inkubation bei 37 °C über Nacht wurden die Kolonien ausgezählt.

Die Ergebnisse der Versuchsauswertung sind als logarithmische Reduktion der Zellzahl der getesteten Organismen in Fig. 3 und Fig. 4 dargestellt. Wie aus Fig. 4 ersichtlich wird, konnte die Wirksamkeit gegenüber Methicillin-resistenten *Staphylococcus aureus* (MRSA) durch Erhöhung des EDTA-Anteils deutlich gesteigert werden.

### Beispiel 5: Nachweis der antimikrobiellen Wirksamkeit im Wundkontaktschichttest nach AATCC 100

Der Test diente dem Wirksamkeitsnachweis der antimikrobiellen Wirkung erfindungsgemäßer Wundauflagen im Kontakttest und erfolgte in Anlehnung an den Standard AATCC 100 nach folgendem Schema:
Eine Übernachtkultur des jeweiligen Mikroorganismus wurde angesetzt durch Inokulation von 10 ml TSB mit einer Einzelkolonie. Die nachfolgende Inkubation fand bei 37°C und 125 rpm auf einem Schüttelinkubator statt. Die Übernachtkulturen wurden auf 1 x 108 CFU / ml eingestellt.

Es wurden erfindungsgemäße Wundkontaktschichten eingesetzt, welche eine Emulsion mit 1,4 Gew.-% antimikrobieller Zusammensetzung enthielten (bezogen auf die ionischen Formen). Die antimikrobielle Zusammensetzung bestand aus 26 Gew.-% EDTA, 46 Gew.-% Ag⁺ und 28 Gew.-% Zn²⁺. Die Wundkontaktschichten wurden jeweils in dreifacher Ausführung getestet. Dazu wurden die Wundkontaktschichten in Petrischalen überführt ( eine Kontaktschicht pro Petrischale) und mit 0,85 %-iger NaCl-Lösung benetzt. Anschließend wurden die Wundkontaktschichten einzeln in 50 ml Falcon Röhrchen eingebracht, mit 10 ml TSB versetzt und mit 100 µl der Übernachtkultur angeimpft, so dass die Sollkonzentration bei 1 x 106 CFU/ml lag.

Die Wundkontaktschichten wurden bei 37°C und 125 rpm auf einem Schüttelinkubator bebrütet. Anschließend wurde 1 ml jeder Probe in neue 50 ml Falcon Röhrchen überführt und 9 ml Neutralisationslösung zugeführt.

Der nachfolgende Schritt erfolgte in einer 96-Well Platte, wobei 200 µl Zelllösung in die Wells der ersten Plattenspalte eingefüllt wurden. In die Wells der Plattenspalten 2 bis 7 wurden 180 µl hinzugeführt. Nun wurde eine 10-fache serielle Verdünnung der Zelllösung angesetzt, indem 20 µl der Lösung in die jeweils nachfolgenden Wellspalten übertragen wurden.

Von jeder Verdünnung wurden 50 µl in zweifacher Ausführung auf TSA-Agarplatten ausplattiert und bei 37 °C über Nacht inkubiert. Am folgenden Tag wurden die Kolonien ausgezählt.

Die Ergebnisse sind in Fig. 5 als Mittelwerte dargestellt.

### Beispiel 6: Bestimmung von Viskosität und Tropfpunkt

Es wurden drei erfindungsgemäße Emulsionen bereitgestellt, wobei jede Emulsion eine andere Konzentration an antimikrobieller Zusammensetzung enthielt. Für den Test wurden Emulsionen mit Konzentrationen von jeweils 0,94 %, 1,4 % sowie 1,94% antimikrobieller Zusammensetzung (bezogen auf die ionischen Formen) gewählt.

Zur Bestimmung des Tropfpunktes wurden die jeweiligen Proben bei Raumtemperatur in den Heizblock einer geeigneten Messvorrichtung gefüllt und dieser mit einer Geschwindigkeit von 1 °C / min erwärmt. Sobald der erste Tropfen der geschmolzenen Probe durch eine Öffnung im Boden des Heizblocks fiel und dabei eine Lichtschranke passierte, wurde die Probentemperatur aufgezeichnet. Dieser Vorgang wurde für jede Emulsion insgesamt drei Mal durchgeführt und die Werte anschließend gemittelt.

Die resultierenden Tropfpunkte können der folgenden Tabelle entnommen werden:

**Tabelle II**

| | | | |
|---|---|---|---|
| Konzentration der antimikrobiellen Zusammensetzung [%] | 0,94 | 1,4 | 1,94 |
| Tropfpunkt der Emulsion [°C] | 38,3 | 38,6 | 39,3 |

Die ermittelten Tropfpunkte liegen allesamt nah an der Körpertemperatur. Durch Einwirkung von Körperwärme steigt die Fließeigenschaft der Emulsion an, wodurch deren Abgabe an die Wunde zunimmt. Dabei wird der Tropfpunkt jedoch nicht erreicht, so dass die Emulsion stabil bleibt.

### Beispiel 7: Substrate für Wundkontaktschichten

Bereitgestellt wurden die folgenden Substrate, welche sich zum Einsatz als Wundkontaktschichten eignen und mit der erfindungsgemäßen Emulsion beschichtet werden können:

### 1) Substrat aus Polyamid:

| | |
|---|---|
| Faseranordnung: | Gewirke in Form eines Tülls |
| Material: | Nylon 6.6 |
| Dicke: | 0,22 mm |
| Fläche: | 10 cm x 10 cm |

### 2) Substrat aus Polyethylenterephthalat:

| | | |
|---|---|---|
| Faseranordnung: | Gewirke | |
| Lineare Dichte: | 76 dtex | (DIN EN ISO 2060) |
| Bruchdehnung: | 26 % | (DIN EN ISO 2062) |
| Festigkeit: | 37 cN/tex | (DIN EN ISO 2062) |
| Dichte: | 1,3 - 1,4 g/cm³ | |

### 3) Substrat mit elementarem, nicht ionischem Silber in der Faserstruktur:

| | |
|---|---|
| Faseranordnung: | Gewirke in Form eines Tülls |
| Material: | 90% Polyamid in Form von Nylon 6.6 und 10 % elementares Silber |
| Dicke: | 0,22 mm |
| Fläche: | 10 cm x 10 cm |
| Flächengewicht: | 33 g / m² |

## Patentansprüche

1. Wundkontaktschicht umfassend ein netzförmiges Substrat, welches mit einer Emulsion teilweise oder vollständig beschichtet ist, wobei die Emulsion eine antimikrobielle Zusammensetzung enthaltend
a) ionisches Silber und / oder Silbernitrat,
b) ionisches Zink und / oder Zinknitrat und / oder Zinksulfat, und
c) EDTA und / oder Tetra-Natrium-EDTA
umfasst.

2. Wundkontaktschicht nach Anspruch 1, **dadurch gekennzeichnet, dass** das netzförmige Substrat mindestens eine der folgenden Fasern enthält oder daraus besteht:
Polyamidfasern, insbesondere Nylonfasern, Polyesterfasern, Baumwollfasern, Viskosefasern.

3. Wundkontaktschicht nach Anspruch 1 oder 2, wobei das netzförmige Substrat als Gewirke oder Tüll vorliegt.

4. Wundkontaktschicht nach einem der vorhergehenden Ansprüche, wobei die antimikrobielle Zusammensetzung
a) 5 bis 50 Gew.-% einer der folgenden Komponenten enthält: ionisches Silber, oder Silbernitrat,
und
b) 3 bis 45 Gew.-% einer der folgenden Komponenten enthält: ionisches Zink oder Zinknitrat oder Zinksulfat und
c) 15 bis 90 Gew.-% EDTA oder Tetra-Natrium-EDTA enthält.

5. Wundkontaktschicht nach einem der vorhergehenden Ansprüche, wobei die antimikrobielle Zusammensetzung 15 bis 30 Gew.-% Tetra-Natrium-EDTA, 35 bis 45 Gew.-% Silbernitrat und 20 bis 40 Gew.-% Zinknitrat enthält.

6. Wundkontaktschicht nach einem der vorhergehenden Ansprüche, wobei die Emulsion 5 bis 40 Gew.-% Wasser enthält.

7. Wundkontaktschicht nach einem der vorhergehenden Ansprüche, wobei die Emulsion 0,5 bis 5 Gew.-% der antimikrobiellen Zusammensetzung enthält.

8. Wundkontaktschicht nach einem der vorhergehenden Ansprüche, wobei die erste und optional auch die zweite Seite des netzförmigen Substrats mit einer Emulsionsmenge von 100 bis 320 g/m² beschichtet ist.

9. Wundkontaktschicht nach einem der vorhergehenden Ansprüche, **dadurch gekennzeichnet, dass** die Emulsion Mono-, Di- und Triglyceride, Fettsäureester, Lanolin, ein Oligomer des Glycerins, Vaseline, Paraffin, synthetisches Wachs oder Mischungen daraus umfasst.

10. Wundkontaktschicht nach Anspruch 9, **dadurch gekennzeichnet, dass** die Emulsion ein Mono-, Di- oder Triglycerid umfasst, welches ein Mono-, Di- oder Triglycerid von Caprylsäure, Caprinsäure, Isostearinsäure, Stearinsäure oder 12-Hydroxystearinsäure ist.

11. Wundkontaktschicht nach einem der vorhergehenden Ansprüche, wobei die Emulsion neben einem möglichen Wasseranteil zusätzlich 3 bis 30 Gew.-% eines weiteren hydrophilen Bestandteils enthält.

12. Wundkontaktschicht nach Anspruch 11, wobei der hydrophile Bestandteil Polymere, Alkohole, Glycerin oder Mischungen daraus umfasst.

13. Wundkontaktschicht nach Anspruch 11 oder 12, wobei der hydrophile Bestandteil ein Polymer ist und wobei das Polymer entweder ein Polysaccharid oder PEG ist.

14. Wundkontaktschicht nach Anspruch 13, wobei das Polymer ein Polysaccharid ist und wobei das Polysaccharid Cellulose, Stärke, modifizierte Stärke, Alginate, Chitosan oder Mischungen daraus umfasst.

15. Wundkontaktschicht nach Anspruch 11 oder 12, wobei der hydrophile Bestandteil ein Alkohol ist und wobei der Alkohol Ethanol, Glycerin, Propylenglykol, Ethylenglykol oder Mischungen daraus umfasst.

16. Wundkontaktschicht nach einem der vorhergehenden Ansprüche, wobei das netzförmige Substrat ein Tüllgewebe ist, das Polyester oder Polyamid enthält und wobei die Emulsion 1,3 bis 1,5 Gew.-% der antibakteriellen Zusammensetzung sowie 26 bis 29 Gew.-% Wasser und darüber hinaus Triglyceride und PEG enthält und wobei die antibakterielle Zusammensetzung 43 bis 49 Gew.-% Silber, 24 bis 30 Gew.-% Zink und 24 bis 28 Gew.-% EDTA enthält.
